# EUROPEAN PATENT APPLICATION

(11) **EP 2 280 025 A2**
(43) Date of publication of application: **02.02.2011**
(21) Application number: 10179425.3
(22) Date of filing: 09.09.2004
(51) Int. Cl.: C07K 11/02, C07K 7/64, A61K 38/15

(54) **New antitumoral compounds**

(30) Priority: 09.09.2003 GB 0321066
(62) Divisional of application: 08100973.0
(71) Applicant: Pharma Mar, S.A.U., 28770 Madrid (ES)
(72) Inventor: Fernandez Donis, Ariadna, 08028 Barcelona (ES); Giralt Lledó, Ernest, 08028 Barcelona (ES); Gracia Cantador, Carolina, 08028 Barcelona (ES); López Rodriguez, Pilar, 08028 Barcelona (ES); Varon Colomer, Sonia, 08028 Barcelona (ES); Cuevas Marchante, Carmen, 28770 Madrid (ES); López Macia, Ángel, 28770 Madrid (ES); Francesch Solloso, Andrés, 28770 Madrid (ES); Jiménez Garcia, José Carlos, 08028 Barcelona (ES); Royo Expósito, Miriam, 08028 Barcelona (ES); Albericio Palomera, Fernando, 08028 Barcelona (ES)
(74) Representative: Spring, Lauren Elizabeth

(57) **Abstract**

New analogues of kahalalide F are provided.

## Description

### FIELD OF THE INVENTION

The present invention is directed to new kahalalide antitumoral compounds, in particular to analogues of Kahalalide F, pharmaceutical compositions containing them and their use as antitumoral, antiviral, antifungal agents and in the treatment of psoriasis.

### BACKGROUND OF THE INVENTION

The kahalalide compounds are peptides isolated from a Hawaiian herbivorous marine species of mollusc, *Elysia rufoscens* and its diet, the green alga *Bryopsis sp..* Kahalalide F is described in Hamann, M et al., J. Am. Chem. Soc., 1993, 115, 5825-5826.

Kahalalide A-G are described in Hamann, M. et al., J. Org. Chem, 1996, 61, 6594-6600: "Kahalalides: bioactive peptides from a marine mollusk Elysia rufescens and its algal diet Bryopsis sp.".

Kahalalide H and J are described in Scheuer P.J. et al., J. Nat. Prod. 1997, 60, 562-567: "Two acyclic kahalalide from the sacoglossan mollusk Elysia rufescens*".*

Kahalalide O is described in Scheuer P.J. et al., J. Nat Prod. 2000, 63(1) 1.52-4: "A new depsipeptide from the sacoglossan mollusk Elysia ornata and the green alga Bryopsis species".

For kahalalide K, see Kan, Y. et al., J. Nat. Prod. 1999 62(8) 1169-72: "Kahalalide K: A new cyclic depsipeptide from the hawaiian green alga Bryopsis species".

For related reports, see also Goetz et al., Tetrahedron, 1999, 55; 7739-7746: "The absolute stereochemistry of Kahalalide F"; Albericio, F. et al. Tetrahedron Letters, 2000, 41, 9765-9769: "Kahalalide B. Synthesis of a natural cyclodepsipeptide"; Becerro et al. J. Chem. Ecol. 2001, 27(11), 2287-99: "Chemical defenses of the sarcoglossan mollusk Elysia rufescens and its host Alga bryopsis sp.".

Of the Kahalalide compounds, kahalalide F is the most promising because of its antitumoral activity. Its structure is complex, comprising six amino acids as a cyclic part, and an exocyclic chain of seven amino acids with a terminal fatty acid group. Originally kahalalide F was reported to have the structure (I).

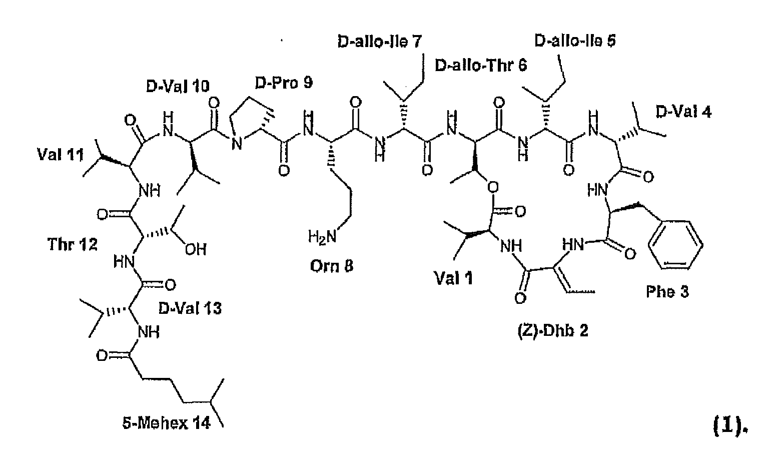

In WO 04035613 there is described the 4(S)-methylhexyl compound with the following formula (II). WO 04035613 is incorporated here in full by specific reference.

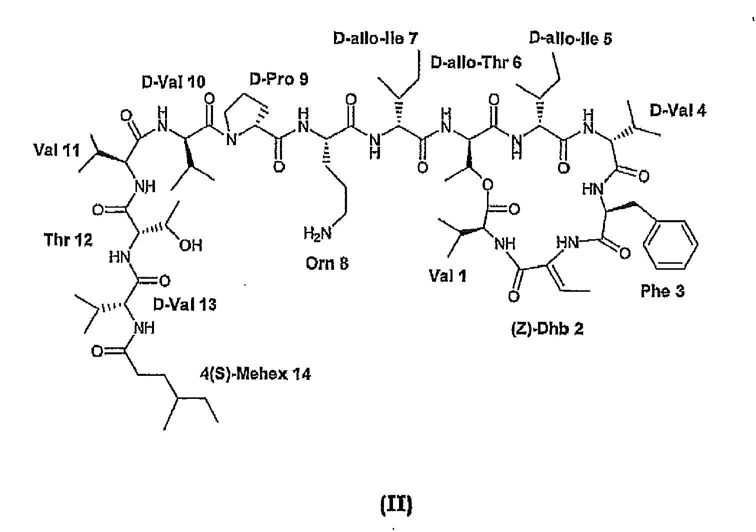

The activity of kahalalide F against *in vitro* cell cultures of human lung carcinoma A-549 and human colon carcinoma HT-29 were reported in EP 610 078. Kahalalide F has also demonstrated to have antiviral and antifungal properties, as well as to be useful in the treatment of psoriasis.

WO 02 36145 describes pharmaceutical compositions containing kahalalide F and new uses of this compound in cancer therapy and is incorporated herein by reference in its entirety.

WO 03 33012 describes the clinical use in oncology of Kahalalide compounds and is incorporated herein by reference in its entirety.

The synthesis and cytotoxic activities of natural and synthetic kahalalide compounds is described in WO 01 58934, which is incorporated herein by reference in its entirety. WO 01 58934 describes the synthesis of kahalalide F and also of compounds with a similar structure in which the terminal fatty acid chain is replaced by other fatty acids.

There is still a need to provide further antitumoral compounds, in particular further kahalalide compounds with improved properties.

### SUMMARY OF THE INVENTION

We have found kahalalide analogue compounds with improved biological activity.

### The present invention is directed to compounds of formula 1

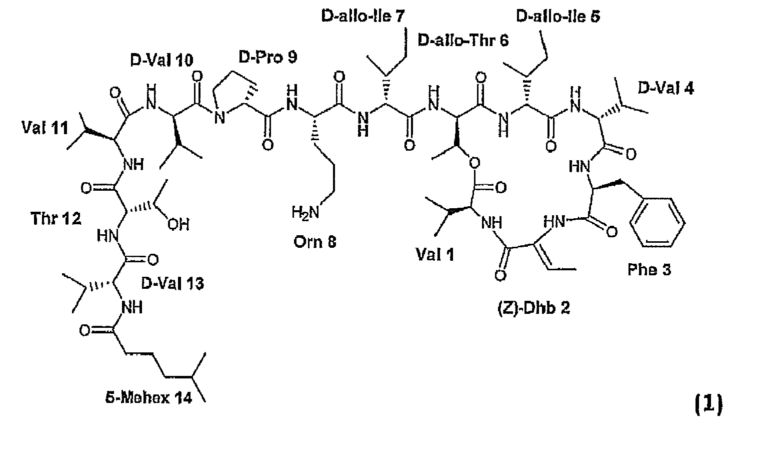

wherein one or more amino acids in the cyclic or exocyclic part have been substituted by other natural or non natural amino acids, have been masked with organic groups or have been removed. The present invention is also directed to compounds of formula 1 wherein the aliphatic terminal acid group has been substituted by other acyl groups or has been removed.

The present invention is also directed to a pharmaceutical composition comprising a compound as previously defined and a pharmaceutically acceptable carrier, vehicle or diluent.

The present invention further provides a method of treating any mammal, notably a human, affected by cancer, viral infection, fungal infection or psoriasis which comprises administering to the affected individual a therapeutically effective amount of a compound as defined above.

The present invention can be employed particularly for treatment of patients with refractory cancers that do not respond favourably to other treatments. In particular, the compositions of this invention can be employed after other chemotherapy has been tried and not worked.

The present invention is particularly directed to the treatment of patients affected with prostate cancer, breast cancer, hepatocellular carcinoma, melanoma, colorectal cancer, renal cancer, ovarian cancer, NSCL cancer, epithelial cancer, pancreatic cancer and tumors that overexpress the Her2/neu oncogene.

In another aspect the present invention is directed to the use of a compound as defined above in the manufacture of a medicament. In a preferred embodiment the medicament is for the treatment of cancer, psoriasis, viral infection or fungal infection.

The invention additionally provides kits comprising separate containers containing a pharmaceutical composition comprising a compound as defined above and a reconstituting agent. Methods of reconstitution are also provided.

### DETAILED DESCRIPTION OF THE INVENTION

We have identified analogues of kahalalide F that show significant improvement in activity with respect to kahalalide F.

### The present invention is directed to compounds of formula 1

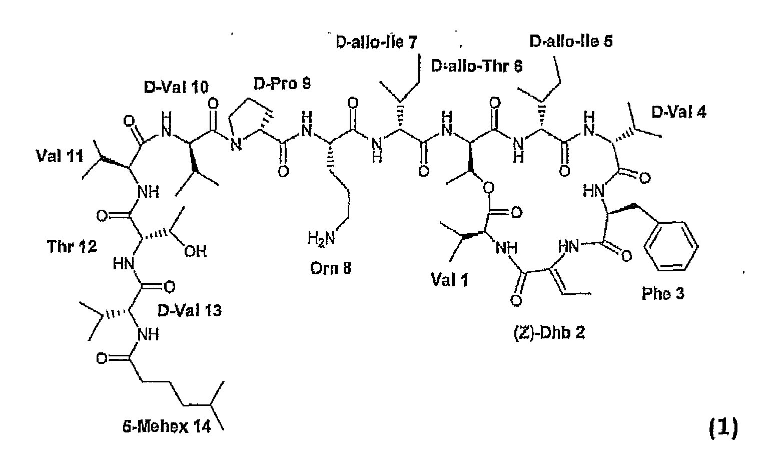

wherein one or more exocyclic or cyclic amino acids have been substituted by other natural or non natural amino acids, have been masked with organic groups or have been removed. The present invention is also directed to compounds of formula 1 wherein the aliphatic methylhexanoic acyl group has been substituted by other acyl groups or has been removed.

### Exocyclic Amino Acids

Preferred compounds of the invention include those of formula 1 wherein one or more amino acids of the exocyclic chain have been substituted by other natural or non natural amino acids, have been masked with organic groups or have been removed.

In particular, preferred compounds include those with 1, 2 or 3 replacement amino acids in the exocyclic chain; and those with 1, 2, 3, 4, 5 or 6 removed amino acids in the exocyclic chain.

Especially preferred are those compounds of formula 1 wherein one exocyclic amino acid has been substituted by another natural or non natural amino acid, and/or has been masked with one or more substituent organic groups. This preference is specially applicable to the L-Orn amino acid in position 8.

Thus, in one aspect of the present invention, there are provided compounds of formula 1 wherein there is not L-Orn at position 8.

The L-Orn can be replaced by D-Orn, or by another natural or non-natural amino acid. For example, the L-Orn can be replaced by a natural amino acid, such as lysine; or a masked natural amino acid, such as arginine or lysine with one or more alkyl, phenyl or oligornethylene subsituents, for example N(Me)₂,N'(Me)₂-Arg, N(Me,Ph),N'(Me)₂-Arg, N(CH₂)₄,N'(Me)₂-Arg, N(CH₂)N'(CH₂)₄-Arg, Nε(Me)₃-Lys.

The L-Orn can be masked. For example, the amino group of the L-Orn may have substituents, notably alkyl substituients that may be further substituted, notably with heterocyclic groups, for example N⁶(CHN(CH₂)₄,N'(CH₂))₄)-Orn; or more complex substituents as in biotinylornithine or Orn(NδTfa).

### Cyclic Amino Acids

Other preferred compounds of the invention include those of formula 1 wherein one or more amino acids of the cyclic chain have been substituted by other natural or non natural amino acids, have been masked with organic groups or have been removed.

In particular, preferred compounds include those with 1, 2 or 3 replacement amino acids in the cyclic chain.

Especially preferred are those compounds of formula 1 wherein one or more amino acids have been substituted by other natural or non natural amino acids, and/or have been masked with organic groups. This preference is specially applicable to the L-Phe amino acid in position 3.

Thus, in one aspect of the present invention, there are provided compounds of formula 1 wherein the L-Phe at position 3 has been replaced or masked.

The L-Phe can be replaced by D-Phe, or by another natural or non-natural amino acid. For example, the L-Phe can be replaced by a natural amino acid, such as tyrosine; an alkyl-substituted ammo acid, such as N-methyltyrosine; an aryl-substituted amino acid, such as 2-amino-3-biphenyl-4-yl-propionic acid, 2-arnino-3-naphthalen-2-ylpropionic acid or aminophenylacetic acid; an heterocyclyl-substituted amino acid, such as 2-amino-3-thiophen-2-ylpropionic acid; or a cyclic amino acid where the amino group is part of a heterocycle, such as 1,2,3,4-tetraisoquinoline-3-carboxilic acid or octahydroisomdole-1-carboxylic acid.

The L-Phe can be masked. For example, the phenyl ring may be partially or fully saturated, and may be substituted with one or more substituents. Substituents for the phenyl ring may be as indicated, preferably halo or nitro. The amino group may have 1 or 2 substituents, notably alkyl such as methyl, especially 1 methyl substituent.

### Terminal Acyl Group

Besides the modification of the amino acids of the exocyclic chain and/or of the cyclic part, there can also be compounds with a modification in the terminal acyl group of the exocyclic chain.

For example, the acyl group can comprise one or more substituents, especially aromatic, heterocyclic or carbocyclic groups which in turn may have one or more subsitutents, for example can be a benzoyl group which may have one or more substituents, a phenylalkanoyl which may have one or more substituents or a cinnamoyl which may have one or more substituents. Additionally, the terminal acyl group can be another fatty acid typically a saturated or unsaturated fatty acid with 3 to 26 carbon atoms, more especially 12 to 20 carbon atoms.

Typical groups for adoption in place of the terminal acyl group in formula 1 include:
- Straight chain alkanoyl with up to 26 carbon atoms and with one or more substituents, especially substituents chosen from optionally substituted cycloalkyl such as 4-methylcyclohexyl; alkyl, especially short chain alkyl such as methyl; optionally substituted aryl especially phenyl such as difluorophenyl; halo such as fluoro up to perfluoro; oxo; amino; or amino.
- Optionally substituted benzoyl such as benzoyl itself, p-methylbenzoyl acid, p-trifluoromethylbenzoyl acid, piperonyloyl.
- Arylalkenoyl group with preferably two carbon atoms in the alkenoyl especially a cinnamoyl group such as p-trifluoromethylcinnamoyl.

Moreover, the acyl group can be replaced by another acyl group, preferably of formula R'CO-. R' is as defined and is suitably alkyl, alkenyl, aryl, heterocyclyl, or carbocyclyl, and may itself be substituted.

Finally, there can be compounds with a unique modification located in the fatty acid of the exocyclic chain, the provision of a 5-methyhexyl terminal group.

### Combined Changes

The possible changes in the exocyclic amino acids, cyclic amino acids and terminal acyl group can be taken in combination.

Thus, other preferred compounds of the invention are those of formula 1 wherein one or more amino acids of the exocyclic chain and/or one or more amino acids of the cyclic part have been modified, as mentioned above and/or the terminal acyl group has been modified.

The compounds where the L-Orn at position 8 has been replaced or masked may adopt other preferred modifications, including a 4(S)-methylhexyl or other group in the terminal group of the sidechain.

The compounds where the L-Phe at position 3 has been replaced or masked may adopt other preferred modifications, including a 4(S)-methylhexyl or other group in the terminal group of the sidechain.

Examples of substituent groups include C₁-C₁₈ alkyl, C₂-C₁₈ alkenyl, c₂-c₁₈ alkynyl, aryl, heterocyclic groups, OR', SR', SOR', SO₂R', NO₂, NHR', N(R')₂, NHCOR', N(COR')₂, NHSO₂R' CN, halogen, C(=O)R', CO₂R', OC(=O)R' wherein each of the R' groups is independently selected from the group consisting of H, OH, NO₂, NH₂, SH, CN, halogen, C(=O)H, C(=O)alkyl, CO₂H, substituted or unsubstituted C₁-C₁₈ alkyl, substituted or unsubstituted C₂-C₁₈ alkenyl, substituted or unsubstituted C₂-C₁₈ alkynyl and substituted or unsubstituted aryl. Suitable halogen substituents in the compounds of the present invention include F, C1, Br and I.

Alkyl groups have a saturated linear or branched hydrocarbon group including, for example, methyl, ethyl, isopropyl, isobutyl, *t*-butyl, heptyl, dodecyl, octadecyl, amyl, 2-ethylhexyl, 2-methylbutyl, 5-methylhexyl, 9-methyl-3-decyl, and the like, particularly alkyl groups which have a single branched methyl group. Suitably the alkyl group is long and has 1 to 20 carbon atoms, more typically 1 to 15 or 1 to 10 carbon atoms, or can be short and has 1 to 6 or 1 to 3 carbon atoms. Long carbon chains are candidates for use in the terminal fatty acid group.

Preferred alkenyl and alkynyl groups in the compounds of the present invention have one or more unsaturated linkages and from 2 to 12 carbon atoms, more preferably 2 to 8 carbon atoms, still more prefereably 2 to 6 carbon atoms, even more prefereably 2, 3 or 4 carbon atoms. The terms alkenyl and alkynyl as used herein refer to both cyclic and noncyclic groups, although straight or branched noncyclic groups are generally more preferred. In a general sense, we include alkylidene within alkenyl, they both being substituents with a double bond.

Suitable aryl groups in the compounds of the present invention include single and multiple ring compounds, including multiple ring compounds that contain separate and/or fused aryl groups. Typical aryl groups contain from 1 to 3 separated or fused rings and from 6 to about 18 carbon ring atoms. Specifically preferred aryl groups include substituted or unsubstituted phenyl, naphthyl, biphenyl, phenanthryl and anthracyl.

Suitable acyl groups include alkanoyl groups which have from 2 to about 12 carbon atoms, more preferably from 2 to about 8 carbon atoms, still more preferably from 2 to about 6 carbon atoms, even more preferably 2 carbon atoms. Other acyl groups include alkenylacyl, alkynylacyl, arylacyl, heterocyclylacyl.

Suitable heterocyclic groups include heteroaromatic and heteroalicyclie groups. Suitable heteroaromatic groups in the compounds of the present invention contain one, two or three heteroatoms selected from N, O or S atoms and include, e.g., coumarinyl including 8-coumarinyl, quinolinyl including 8-quinolinyl, pyridyl, pyrazinyl, pyrimidyl, furyl, pyrrolyl, thienyl, thiazolyl, oxazolyl, imidazolyl, indolyl, benzofuranyl and benzothiazol. Suitable heteroalicyclic groups in the compounds of the present invention contain one, two or three heteroatoms selected from N, O or S atoms and include, e.g., tetrahydrofuranyl, tetrahydropyranyl, piperidinyl, morpholino and pyrrolindinyl groups.

The groups above mentioned may be substituted at one or more available positions by one or more substituents.

The natural amino acids include alanine, glycine, valine, leucine, isoleucine, phenylalanine, tyrosine, tryptophan, methionine, cysteine, aspartate, asparagine, glutamatic acid, glutamine, lysine, arginine, proline, serine, threonine, histidine and hydroxyproline

We also refer to the WO 0158934 incorporated herein in full by specific reference. That earlier text gives guidance which is applicable to the compounds of this invention, and we import the definitions for adoption in the compounds of this invention.

Abbreviations used in the following description for amino acids and the designations of peptides follow the rules of the IUPAC-IUB Commission of Biochemical Nomenclature in J. Biol. Chem., 1972, 247, 977-983.

### The following additional abbreviations are used:

| Alloc | allyloxycarbonyl |
|---|---|
| *N*(CH₂)₄,*N*'(CH₂)4-Arg, | 2-amino-5-[(dipyrrolidin-1-yl-methylene)-aminol]-pentanoic acid |
| AM | maslinic acid |
| AO | oleanolic acid |
| Bip | 2-amino-3-biphenyl-4-yl-propionic acid |
| Boc | *tert*-butyloxycarbonyl |
| BTFFH | bis(tetramethylene)fluoroformamidinium hexafluorophosphate |
| *t*-Bu | *tert*-Butyl |
| Bza-OH | benzoic acid |
| Cha | cyclohexylalanine or 2-amino-3-cyclohexyl- |
| | propionic acid |
| *p*-CF₃Bza-OH | 4-trifluoromethylbenzoic acid |
| *p*-CF₃BzAc-OH | 3-(4-Trifluoromethylbenzyl)acetic acid |
| *p*-CF₃Cinn-OH | 3-(4-trifluoromethylbenzyl)acrylic acid |
| C1-TrtC1-resin | 2-chlorotrityl chloride-resin |
| Dha | 2-aminoacrylic acid or didehydroalanine |
| Z-Dhb | α,β-didehydro-a-aminobutyric acid |
| DIPEA | N,N-diisopropylethylamine |
| DMF | *N*,*N*-dimethylformamide |
| EDCHC1 | 1-ethyl-3-(3'- |
| | dimethylaminopropyl)carbodiimide |
| | hydrochloride |
| Fmoc | 9-fluorenylmethoxycarbonyl |
| 6,6-dFHep-OH | 6,6-difluoro-heptanoic acid |
| 3,5-dFPhAc-OH | (3,5-difluorophenyl)acetic acid |
| 4-GuBut-OH | 4-guanidinobutyric acid |
| GI50 | growth inhibition at 50% |
| HAPyU | *O*-(7-azabenzotriazol-1-yl)-1,1,3,3- |
| | bis(tetramethylene)uronium |
| | hexafluorophosphate |
| HATU | *O*-(7-azabenzotriazol-1-yl)-1,1,3,3-tetrarnethyluronium hexafluorophosphate |
| hCh | homocyclohexylalanine or 2-amino-4-cyclohexylbutyric acid |
| Hep-OH | heptanoic acid |
| HOAc | acetic acid |
| HOAt | 1-hydroxy-7-azabenzotriazole (3-hydroxy-3*H*-1,2,3-triazolo-[4,5-b]pyridine) |
| HOBt | 1-hydroxybenzotriazole |
| Icos-OH | icosanoic acid |
| LC50 | lethal concentration at 50% |
| Lit-OH | litocholic acid |
| MeHex-OH | methyhexanoic acid |
| M_{2A} | [(tetramethylene)-1*H*-1,2,3-trazolo[4,5- |
| | b]pyidino-1-ylmethylene]-*N*- |
| | methylmethanaminium hexafluorophosphate |
| (c/t-4Me-cHexa)-OH | (cis/trans)-4-methylcyclohexanecarboxylic |
| | acid |
| *p*-MeBza-OH | 4-Methyl-benzoic acid |
| *N*(Me)₂,*N*'(Me)₂-Arg | 2-Amino-5-(*N*',*N*',*N*",*N*"-. |
| | tetramethylguanidino)pentanoic acid |
| *N*(CH₂)₄,*N'*(Me)₂-Arg | 2-amino-5-[(dimethylamino-pyrrolidin-1-yl- |
| | methylene)-amino]-pentanoic acid |
| MeOH | methanol |
| *N*(Me,Ph),*N*'(Me)₂-Arg | 2-amino-5-(*N*',*N*',*N*'*-*trimethyl-*N*"-phenyl- |
| | guanidino)pentanoic acid |
| NMM | *N*-methylmorpholine |
| Mst-OH | myristic acid or tetradecanoic acid |
| NaI | 2-amino-3-naphthalen-2-yl-propionic acid |
| Oct-OH | octanoic acid |
| 6-OHep-OH | 6-oxo-heptanoic acid |
| Oic | octahydro-isoindole-1-carboxylic acid |
| [*N*δ(CH-*N*(CH₂)₄- | 2-amino-5-[(dipyrrolidin-1-yl-methyl)- |
| *N*'(CH₂)₄)-Orn | amino]pentanoic acid |
| Phf-OH | perfluoroheptanoic acid |
| Phg | phenylglycine or aminophenylacetic acid |
| (5R)-Ph-Pro | 5-(R)-phenyl-pirrolidine-2-carboxilic acid |
| Pip | pipecolic acid |
| Pipe-OH | benzo[1,3]dioxole-5-carboxilic acid |
| SPS | solid-phase synthesis |
| Tfa | trifluoroacetyl |
| TFA | trifluoroacetic acid |
| TFAA | trifluoroacetic anhydride |
| TGI | total growth inhibition |
| Thi | ala-[3-(2-thienyl)] or 2-amino-3-thiophen-2-yl- |
| | propionic acid |
| Tic | 1,2,3,4-tetraisoquinoline-3-carboxilic acid |
| Und-OH | undecanoic acid. |

Amino acid symbols denote the L-configuration unless stated otherwise.

Examples of compounds of this invention include those of formula 1 wherein there is:
- Glu or Lys instead of L-Orn in position 8;
- Gly, Phe, Ala, Leu, D-Val, Pro, Gln, Orn, Thr or Glu instead of L-Val in position 11;
- Val or D-Thr instead of L-Thr in position 12;
- Gly, D-Ala, D-Leu, D-Phe, D-Pro, Val, D-Glu, D-Gln or D-Thr instead of D-Val in position 13;
- hCh instead of L-Val in position 11 and/or D-Cha instead of D-Val in position 13;
- Ala, Gly, Leu, Pro, Glu, Orn or Gln instead of L-Thr in position 12 and absence of amino acid in position 13;
- D-Ile or D-Val instead of D-allo-Ile in position 7;
- D-Om instead of L-Orn in position 8 and L-Pro instead of D-Pro in position 9;
- D-Cys instead of D-allo-Ile in position 7 and L-Cys instead of L-Val in position 11; or
- D-Cys or D-homo-Cys instead of D-allo-Ile in position 7 and D-Cys or D-homo-Cys instead of D-Val in position 10.
- Icos, (c/t)-4-Me-cHexa, Und, (4R)-MeHex, (4RS)-MeHex, (4S)-MeHex, Oct, p-MeBza, Bza, p-CF₃Bza, 3,5-dFPhAc, Pipe, p-CF₃Cinn, p-CFsFhAc, Pfh, 6-OHep, 6,6-dFHep, 4-GuBut, AM, AO or C(=N(CH₃)₂) instead of 5-MeHex in position 14, and, optionally, Lys instead of L-Orn in position 8;
- Pro, D-Pip, D-Tic or (5R)-Ph-Pro instead of D-Pro in position 9 and (4S)-MeHex instead of 5-MeHex in position 14;
- N(Me)₂,N'(Me)2-Arg, N(Me,Ph),N'(Me)₂-Arg, N(CH₂)₄,N'(Me)₂-Arg, N(CH₂)₄,N'(CH₂)₄-Arg, Nδ(CHN(CH₂)₄,N'(CH₂)₄)-Orn, N^{e}(Me)₃-Lys, Orn(NδTfa) or Om(Biot) instead of L-Orn in position 8, and, optionally (4S)-MeHex instead of 5-MeHex in position 14 and, optionally, Thr(OTfa) instead of L-Thr in position 12;
- Thr(OTfa) instead of L-Thr in position 12 and (4S)-MeHex or Lit(OTfa) instead of 5-MeHex in position 14;
- N-(Hep)₂-D-Val instead of D-Val in position 13 and there is absence of 5-MeHex in position 14; or
- absence of amino acids in position 11, 12 and 13, and, optionally, Mst instead of 5-MeHex in position 14.
- Dha or E-Dhb instead of Z-Dhb in position 2;
- D,L-Ser, Gly or Aib instead of Thr in position 2, being the analogues hydrogenated;
- D-Val instead of L-Val in position 1;
- Trp instead of L-Phe in position 3; or
- D-Thr or D-Ser instead of D-alo-Thr in position 6.
- hCh, Phe (3,4-Cl₂), Phe (F₅), Phe (4-I), Phe (4-NO₂), Phe (4-F), Tyr (Me), Thi, Tic, Tyr, Oic, NMePhe, Phe (2-Cl), Phe (3-Cl), Phe (4-Cl), Phe (3,4-F₂), NaI, Bip or Phg instead of L-Phe in position 3, and, optionally, (4S)-MeHex or p-CF₃Cinn instead of 5-MeHex in position 14.
- D-Val or D-Cha instead of D-alo-Ile in positions 5 and 7 and, optionally, D-Cha instead of D-Val in position 4; or
- D-Val instead of L-Val in position 1, D-Phe instead of L-Phe in position 3, Val instead of D-Val in position 4, L-alo-Ile instead of D-alo-Ile in position 5, L-alo-Thr instead of D-alo-Thr in position 6, L-alo-Ile instead of D-alo-Ile in position 7, D-Orn instead of L-Orn in position 8, L-Pro instead of D-Pro in position 9, L-Val instead of D-Val in position 10, D-Val instead of L-Val in position 11, D-Thr instead of L-Thr in position 12 and L-Val instead of D-Val in position 13.

As appropriate, these suggested changes may be taken in combination.

The present invention also encompass the pharmaceutically acceptable salts, prodrugs, tautomers, and solvates, thereof.

The compounds of the present invention have asymmetric centers and therefore exist in different enantiomeric and diastereomic forms. This invention relates to the use of all optical isomers and stereoisomers of the compounds of the present invention, and mixtures thereof, and to all pharmaceutical compositions and methods of treatment that may employ or contain them.

The compounds of the invention may be in crystalline form either as free compounds or as solvates (e.g. hydrates) and it is intended that both forms are within the scope of the present invention. Methods of solvation are generally known within the art.

The present invention also includes the compounds of the present invention, and the pharmaceutically acceptable salts thereof, wherein one or more hydrogen, carbon or other atoms are replaced by isotopes thereof. Such compounds may be useful as research and diagnostic tools in metabolism pharmacokinetic studies and in binding assays.

As used herein, the compounds of this invention, including the compounds of formula 1, are defined to include pharmaceutically acceptable derivatives or prodrugs thereof. A "pharmaceutically acceptable derivative or prodrug" means any pharmaceutically acceptable salt, ester, salt of an ester or other derivative of a compound of this invention that, upon administration to a recipient, is capable of providing (directly or indirectly) a compound of this invention or a metabolite or residue thereof. Particularly favoured derivatives and prodrugs are those that increase the bioavailability of the compounds of this invention when such compounds are administered to a patient (e.g., by allowing an orally administered compound to be more readily absorbed into the blood), enhance delivery of the parent compound to a given biological compartment, increase solubility to allow administration by injection, alter metabolism or alter rate of excretion.

Salts of the compounds of the present invention may comprise acid addition salts derived from a nitrogen in the compound of formula 1 or 2. The therapeutic activity resides in the moiety derived from the compound of the invention as defined herein and the identity of the other component is of less importance although for therapeutic and prophylactic purposes it is, preferably, pharmaceutically acceptable to the patient. Examples of pharmaceutically acceptable acid addition salts include those derived from mineral acids, such as hydrochloric, hydrobromic, phosphoric, metaphosphoric, nitric and sulphuric acids, and organic acids, such as tartaric, acetic, trifluoroacetic, citric, malic, lactic, fumaric, benzoic, glycolic, gluconic, succinic and methanesulphonic and arylsulphonic, for example p-toluenesulphonic, acids. Preferred salts include the trifluoroacetate salt and the hydrochloride salt.

The compounds of the present invention can be prepared according to the synthetic process described in WO 01 58934, or according to the improved process as described herein. Therefore also encompassed by the invention is a process to prepare a compound according to formula 1.

The key steps of the optimized process for a more economical and safe synthesis of kahalalide F and its analogues are: (i) partial incorporation of Fmoc-D-Val-OH onto the chlorotritylchloro-polystyrene resin for a initial loading of 0.5 mmol/g; (ii) use as coupling method of DIPCDI-HOBt, instead of HATU-DIPEA, for the sequential incorporation of the protected amino acids and aliphatic carboxylic acids; (iii) cyclization step with DIPCDI/HOBt/DIPEA in CH₂Cl₂; these conditions avoids two side reaction: epimerisation of the Val residue, which is involved in the activation, and trifluoroacetylation of the Phe or its replacement; (iv) use of sodium diethyl-dithiocarbamate after removing Alloc to avoid presence of Pd (0) in the final product.

### The synthesis is preferably a solid phase synthetic process.

The preferred embodiment of the synthetic process of the present invention is best represented in the Scheme 1, which is directed to the formation of the target compounds.

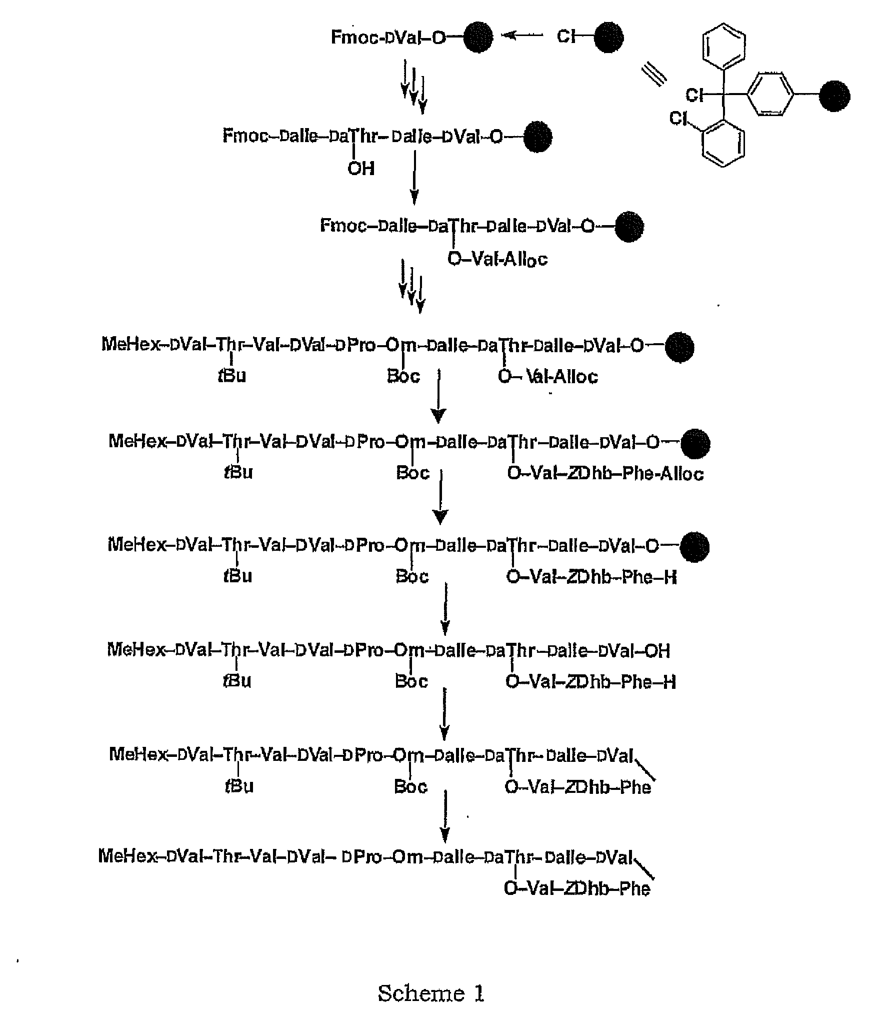

As shown above in Scheme 1, the preferred process for the synthetic formation of analogues of kahalalide F is based in a solid-phase approach, see for example Lloyd-Williams, P., et al. Chemical Approaches to the Synthesis of Peptides and Proteins. CRC Press, Boca Raton (FL), 1997 and followed with modifications of the method already described for the preparation kahalalide F and some of its analogues (WO 01 58934).

The process of Scheme 1 comprises the sequential steps of:
(a) incorporating an Fmoc-DVal-OH onto a chlorotrityl chloro resin, forming an ester bond;
(b) elongating the peptidic chain with three amino acids [DaIle, DaThr (free OH), DaIle) using a Fmoc/*t*Bu strategy;
(c) incorporating [Val(1)] using an Alloc/*t*Bu strategy;
(d) elongating the peptidic chain with the remaining amino acids and aliphatic carboxylic acids using a Fmoc/*t*Bu strategy;
(e1) incorporating the dipeptide Alloc-Phe-*Z*Dhb-OH, which has been combined and dehydrated in solution;
(e2a) elongating the peptidic chain with two amino acids, preferably Thr and Phe. The OH of Thr is unprotected and the amino group of Phe, or its replacement, is protected with Fmoc or preferably with Alloc; in some cases if it is protected with Fmoc, this is removed and Alloc is introduced in solid-phase; ,
(e2b) dehydrating in solid-phase to give the didehydropeptide;
(f) removing the Alloc/Fmoc group of Phe, or of its replacement, while the peptide is still anchored to the solid support;
(g) cleaving the side-chain protected peptide from the solid support;
(h) cyclizing the peptide in solution;
(i) removing TFA labile side chain protecting groups.
(j) Further modifications of functional(s) group(s) in solution phase.

Therefore the process can be conducted as follows:
Fmoc-DVal-OH is incorporated preferably to a chlorotritylpolystyrene resin, see Barlos, K.; Gatos, D.; Schäfer, W. Angew. Chem. Int. Ed. Engl. 1991, 30, 590-593, in the presence of DIPEA keeping the level of substitution of aprox. 0.5 mmol/g. The use of higher loadings brings the presence of terminated peptides in the final product, see Chiva, C.; Vilaseca, M.; Giralt, E.; Albericio, F. J. Pept. Sci. 1999, 5, 131-140.

### All solvent ratios are volume/volume unless stated otherwise.

Removal of the Fmoc group can be carried out with piperidine-DMF (2:8, v/v) (1 x 2 min, 2 x 10 min). Couplings of Fmoc-aa-OH (4-5 equiv) and the acids at the 14-position can be carried out with DIPCDI-HOBt (equimolar amounts of each one respect to the carboxylic component) or PyBOP-DIPEA (equimolar amount of PyBOP and double amount of DIPEA) in DMF or DMF-Toluene (1:1) for 90 min. After the coupling ninhydrin or chloranil tests are carried out and if it is positive the coupling is repeated in the same conditions, otherwise the process is continued. Washings between deprotection, coupling, and, again, deprotection steps can be carried out with DMF (5 x 0.5 min) and CH₂Cl₂ (5 x 0.5 min) using for example each time 10 mL solvent/g resin.

Incorporation of Alloc-Val-OH (5 equiv) can be carried out with equimolar amount of DIPCDI and 10% of DMAP. This coupling is repeated at least twice.

Removal of Alloc group can be carried out with Pd(PPh₃)₄ (0.1 equiv) in the presence of PhSiH₃ (10 equiv), see Gómez-Martinez, P.; Thieriet, N.; Albericio, F.; Guibé, F. J. Chem. Soc. Pekin I 1999, 2871-2874, and washing the resin with sodium diethyldithiocarbamate in DMF 0.02 M (3 x 15 min).

The dipeptide Alloc-Phe-ZDhb-OH (4 equiv), which was prepared in solution from Alloc-Phe-OH and H-Thr-O*t*Bu with EDC·HCl, and posterior dehydration and treatment with TFA, can be coupled with DIPCDI-HOAt (4 equiv of each) for 5 h to overnight. The use of other coupling reagents based in HOBt, such as HBTU or DIPCDI-HOBt, has led to incomplete incorporations of the dipeptide.

Dehydration can be carried out in solid-phase with EDC·HCl (water soluble carbodiimide, 20 equiv) in the presence of CuCl (12 equiv) in CH₂Cl₂-DMF (9:1) for 7 days. EDC·HCl/CuCl has been used by dehydration in solution of a residue of Thr in a fragment of Nisin (Fukase, K.; Kitazawa, M.; Sano, A.; Shimbo, K.; Horimoto, S.; Fujita, H.; Kubo, A.; Wakamiya, T.; Shibe, A. Bull. Chem. Soc. Jpn. 1992, 65, 2227-2240) and in solid-phase for the preparation of didehydropeptides from Thr, Ser, and phenylserine (Royo, M.; Jiménez, J.C.; López-Macià, A.; Giralt, E.; Albericio, F. Eur. J. Org. Chem. 2001, 45-48).

Cleavage of the protected peptide from the resin can be accomplished by TFA-CH₂Cl₂ (1:99) (5 x 30 sec).

Cyclization step can be carried out with DIPCDI/HOBt/DIPEA in CH₂Cl₂. These conditions avoid two side reactions: epimerisation of the Val residue, which is involved in the activation, and trifluoroacetylation of the Phe or its replacement.

Final deprotection can be carried out with TFA-H₂O (95:5) for 1 hour.

It will be appreciated that the particular choice of protecting groups is not critical, and other choices are widely available. For example, Bzl type groups can replace tBu/Boc; Boc instead of Fmoc; Fmoc instead of Alloc; Wang resin instead of chlorotrityl.

### Further detail on the synthesis is given in the examples.

The process of this invention can be carried out from starting materials in an enantio-, stereocontrolled and fast manner, taking advantages of the solid-phase synthetic methodology, where the molecule in construction is bounded to an insoluble support during all synthetic operations.

Pharmaceutical formulations of the compounds of the invention may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier (s) or excipient (s).

Preferably pharmaceutical compositions of the compounds of the invention include liquid (solutions, suspensions or emulsions) with suitable composition for intravenous administration, and they may contain the pure compound or in combination with any carrier or other pharmacologically active compounds. Further guidance concerning the pharmaceutical compositions can be found in WO 02 36145 which is incorporated herein by reference in its entirety.

Thus, a combination of a non-ionic surfactant and an organic acid is suited for use with a bulking agent to give a lyophilised form of a compound of the invention suited for reconstitution. Reconstitution is preferably effected with a mix of emulsifying solubiliser, alkanol and water.

The lyophilised composition preferably comprises mainly the bulking agent, such as at least 90 % or at least 95 % bulking agent. Examples of bulking agents are well known and include sucrose and mannitol. Other bulking agents can be employed.

The non-ionic surfactant in the lyophilised composition is preferably a sorbitan ester, more preferably a polyethylene sorbitan ester, such as a polyoxyethylene sorbitan alkanoate, especially a polyoxyethylene sorbitan mono-oleate, for example polysorbate 80. The non-ionic surfactant typically comprises a few % of the composition, such as 0 to 5 % of the composition, for instance 2 to 3 or 4 % of the composition.

The organic acid in the lyophilised composition is typically an aliphatic acid, preferably a hydroxycarboxylic acid and more preferably a hydroxypolycarboxylic acid, notably citric acid. The organic acid typically comprises a few % of the composition, such as 0 to 5 % of the composition, for instance 2 to 3 or 4 % of the composition.

The amount of the compound of the invention in the lyophilised composition is typically less than 1 %, or often less than 0.1 %, of the mix. A suitable amount is in the range 50 to 200 µg, say about 100 µg, per 100 mg of composition.

The emulsifying solubiliser for the reconstituting agent suitably comprises an polyethylene glycol ester, notably an ester of a fatty acid, more preferably a PEG oleate such as PEG-35 oleate. The emulsifying solubiliser is suitably 0 to 10 % of the reconstituting agent, typically about 3 to 7 %, say about 5 %. The alkanol is usually ethanol, and is suitably 0 to 10 % of the reconstituting agent, typically about 3 to 7 %, say about 5 %. The remainder of the reconstituting agent is water, and gives a reconstituted solution suited for intravenous injection.

Further dilution of the reconstituted solution with 0.9 % saline may be appropriate for infusion of the kahalalide compound. Suitable infusion equipment preferably includes a glass container, rather than one of polyethylene. Tubing is preferably of silicone.

The preferred reconstituting agent then comprises 2 to 7 %, say about 5 %, emulsifying solubiliser; 2 to 7 %, say about 5 %, alcohol; and remainder water.

The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use.

The invention additionally provides kits comprising separate containers containing the lyophilised composition and the reconstituting agent. Methods of reconstitution are also provided.

Administration of the compounds or compositions of the present invention is by intravenous infusion. Infusion times of up to 72 hours can be used, more preferably 1 to 24 hours, with either about 1 or about 3 hours most preferred. Short infusion times which allow treatment to be carried out without an overnight stay in hospital are especially desirable. However, infusion may be around 24 hours or even longer if required.

The administration is performed in cycles, in the preferred application method, an intravenous infusion of a compound of the invention is given to the patients the first week of each cycle, the patients are allowed to recover for the remainder of the cycle. The preferred duration of each cycle is of either 1, 3 or 4 weeks; multiple cycles can be given as needed. In an alternative dosing protocol, the compound of the invention is administered for say about 1 hour for 5 consecutive days every 3 weeks. Other protocols can be devised as variations.

Dose delays and/or dose reductions and schedule adjustments are performed as needed depending on individual patient tolerance of treatments, in particular dose reductions are recommended for patients with higher than normal serum levels of liver transaminases or alkaline phosphatase.

In one aspect, the present invention provides a method for treating a human patient afflicted with cancer, comprising administering to said patient a compound of the invention at a dose below 1200 mcg/m2/day, preferably below 930 mcg/m2/day and more preferably below 800 mcg/m2/day. Suitably the dose is at least 320 mcg/m2/day. Preferably the dose is in the range of 400-900 mcg/m2/day, preferably 500-800 mcg/m2/day, more preferably 600-750 mcg/m2/day. Especially preferred are doses of about 650-700 mcg/m2/day.

In a further aspect the invention provides a method for treating a human patient afflicted with cancer, comprising administering to said patient a compound of the invention daily during 5 days at a dose below 930 mcg/m2/day, followed by a resting period of from 1 to 4 weeks in which the kahalalide compound is not administered. The dose is preferably 650-750 mcg/m2/day, more preferably about 700 mcg/m2/day. The infusion time is preferably between 1 and 24 hours, more preferable between 1 and 3 hours. Especially preferred is an infusion time of about 1 or about 3 hours. The resting period is preferably 2-3 weeks, more preferably about 2 weeks.

The present invention also provides a method for treating a human patient afflicted with cancer, comprising administering to said patient a compound of the invention once weekly at a dose below 800 mcg/m2/day. The dose is preferably 600-700 mcg/m2/day, more preferable 650 mcg/m2/day. The infusion time is preferably between 1 and 24 hours, more preferably between 1 and 3 hours.

Although guidance for the dosage is given above, the correct dosage of the compound will vary according to the particular formulation, the mode of application, and the particular situs, host and tumour being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

The present invention is particularly directed to the treatment of patients affected with prostate cancer, breast cancer, hepatocellular carcinoma, melanoma, colorectal cancer, renal cancer, ovarian cancer, NSCL cancer, epithelial cancer, pancreatic cancer and tumors that overexpress the Her2/neu oncogene. Most preferably it is directed to the treatment of hepatocellular cancer, melanoma, breast cancer, pancreatic cancer and prostate cancer.

The present invention is also directed to a method of treating a skin disease involving hyperproliferation of dermis cells in a mammal which comprises administering to the mammal an effective, non-toxic amount of a compound of the invention. The skin disease is preferably psoriasis. The present invention is preferably directed to the treatment of human patients affected with psoriasis, in particular severe psoriasis.

The compounds and compositions of this invention may be used with other drugs to provide a combination therapy. The other drugs may form part of the same composition, or be provided as a separate composition for administration at the same time or a different time. The identity of the other drug is not particularly limited, although combination with other chemotherapeutic, hormonal or antibody agents is envisaged. The amounts of the compound of the invention and the other pharmaceutically active agent or agents and the relative timings of administration will be selected in order to achieve the desired combined therapeutic effect.

### EXAMPLES

### General Procedures.

Cl-TrtCl-resin, Protected Fmoc-amino acid derivatives, HOBt, HOAt were from ABI (Framingham, MA), Bachem (Bubendorf, Switzerland), NovaBiochem (Läufelfingen, Switzerland), 4-MeHex derivatives from Narchem, HATU and other guanidylation derivatives were from ABI (Framingham, MA) or prepared as in del Fresno, M.; El-Faham, A.; Carpino, L.A.; Royo, M.; Albericio, F. Organic Lett., 2000, 2, 3539-3542.

Alloc-amino acids were prepared essentially as described by Dangles *et al*. see Dangles, O.;Guibé, F.; Balavoine, G.; Lavielle, S.; Marquet. A. J. Org. Chem. 1987, 52, 4984-4993 and Alloc-*Z*-Dhb-Phe-OH and Kahalalide F as described in WO 01 58934, DIPEA, DIPCDI, EDC·HCl, Piperidine, TFA were from Aldrich (Milwaukee, WI). DMF and CH₂Cl₂ were from SDS (Peypin, France). Acetonitrile (HPLC grade) was from Scharlau (Barcelona, Spain). All comercial reagents and solvents were used as received with exception of CH₂Cl₂, which was passed through a alumina column to remove acidic contaminants.

Solid-phase syntheses were carried out in polypropylene syringes (10-50 mL) fitted with a polyethylene porous disc. Solvents and soluble reagents were removed by suction. Removal of the Fmoc group was carried out with piperidine-DMF (2:8, v/v) (1 x 2 min, 2 x 10 min). Washings between deprotection, coupling, and, again, deprotection steps were carried out with DMF (5 x 0.5 min) and CH₂Cl₂ (5 x 0.5 min) using each time 10 mL solvent/g resin. Peptide synthesis transformations and washes were performed at 25 °C. Syntheses carried out on solid-phase were controlled by HPLC of the intermediates obtained after cleaving with TFA-H₂O (1:99) for 1 min an aliquot (aprox. 2 mg) of the peptidyl-resin. HPLC reversed phase columns [Nucleosil-C₁₈ 4,6 x 250 mm, 10 µm (column A); Nucleosil-C₄ 4,6 x 250 mm, 10 µm (column B) were from Sharlau (Spain); Symmetry™ C₁₈ 4,6 x 150 mm, 5 µm (column C); Symmetry300^{TM} C₁₈ 4,6 x 50 mm, 5 µm (column D) were from Waters (Ireland) and Zorbax SB C₁₈ 4,6 x 150 min, 3.5 µm (column E) was from Agilent (USA)]. Analytical HPLC was carried out on a Waters instrument comprising two solvent delivery pumps (Waters 1525), automatic injector (Waters 717 autosampler), dual wavelength detector (Waters 2487), and system controller (Breeze V3.20) and on a Agilent 1100 instrument comprising two solvent delivery pumps (G1311A), automatic injector (G1329A), DAD (G1315B). UV detection was at 215 or 220 nm, and linear gradients of CH₃CN (+0.036% TFA) into H₂O (+0.045% TFA) were run in the following conditions:

**Table I**

| Chromatographic Conditions | Flow (mL/mln) | Gradient (% of CH₃GN) | Runing Time (min) |
|---|---|---|---|
| A | 1.0 | 30 to 100 | 30 |
| B | 1.0 | 40 to 60 | 15 |
| C | 1.0 | 45 to 60 | 8 |
| D | 1.0 | 40 to 70 | 8 |
| E | 1.0 | 46 to 70 | 8 |
| F | 1.0 | 40 to 70 | 15 |
| G | 1.0 | 40 to 65 | 15 |
| H | 1.0 | 10 to 100 | 30 |
| I | 1.0 | 45 to 65 | 16 |
| J | 1.0 | 40 to 70 | 15 |
| K | 1.0 | 55 to 75 | 15 |
| L | 1.0 | 40 to 100 | 15 |
| M | 1.0 | 50 to 100 | 8 |
| N | 1.0 | 35 to 60 | 15 |
| O | 1.0 | 50 to 100 | 30 |
| P | 1.0 | 50 to 100 | 15 |
| Q | 1.0 | Isocratic at 45 | 15 |
| R | 1.0 | 30 to 100 | 15 |
| S | 1.0 | 20 to 100 | 8 |
| T | 0.6 | 35 to 90 | 25 |
| U | 0.7 | 40 to 70 | 50 |
| V | 0.8 | 10 to 48 in 15 min and isocratic 30 min | 45 |
| W | 1.0 | 10 to 70 | 50 |
| X | 1.0 | 10 to 48 in 15 min and isocratic 30 min | 45 |
| Y | 0.8 | 10 to 60 | 50 |
| Z | 0.7 | 15 to 70 | 55 |
| AB | 0.8 | 10 to 60 | 55 |
| AC | 0.8 | 30 to 60 | 45 |
| AD | 0.8 | 10 to 48 in 10 min and isocratic 30 min | 40 |
| AE | 1.0 | 45 to 90 | 25 |
| AF | 1.0 | 40 to 100 | 8 |

MALDI-TOF and ES-MS analysis of peptide samples were performed in a PerSeptive Biosystems Voyager DE RP, using DHB matrix, and in a Waters Micromass ZQ spectrometer and in an Agilent Ion Trap 1100 Series LC/MSDTrap. Peptide-resin samples were hydrolyzed in 12 N aqueous HCl-propionic acid (1:1), at 155 °C for 1-3 h and peptide-free samples were hydrolyzed in 6 N aqueous HCl at 155 °C for 1 h. Subsequent amino acid analyses were performed on a Beckman System 6300 autoanalyzer. ¹H-NMR spectroscopy [1H, NOESY, TOCSY at (278K)] was performed on a Varian Unity Plus (500 MHz). Chemical shifts (δ) are expressed in parts per million downfield from TMS. Coupling constants are expressed in hertz.

The names of the analogues are named relative to the 5-methyhexyl isomer of Kahalalide F of formula (I), indicating between square brackets the modified residue; the suffix "no" indicates the elimination of the natural residue from the sequence.

### Example 1

**(4S)-MeHex-D-Val-ThrVal-D-Val-D-Pro-Orn-D-*allo*-Ile-cyclo[D-allo-Thr-D-*allo*-Ile-D-Val-Phe-(Z)Dhb-Val], [(4S)-MeHex¹⁴]-Kahaialide** F (Compound 1)

### Step 1

### H-D-Val-G-TrtCl-resin.

Cl-TrtCl-resin (1 g, 1.64 mmol/g) was placed in a 20 mL polypropylene syringe fitted with a polyethylene filter disk. The resin was then washed with CH₂Cl₂ (5 x 0.5 min), and a solution of Fmoc-D-Val-OH (238 mg, 0.7 mmol, 0.7 equiv) and DIPEA (0.41 mL) in CH₂Cl₂ (2.5 mL) was added, and the mixture was stirred for 15 min, when extra DIPEA (0.81 mL, total 7 equiv, 7 mmol) and the mixture stirred for 45 min. The reaction was terminated by addition of MeOH (800 µL), after a stirring of 10 min. The Fmoc-D-Val-O-TrtCl-resin was subjected to the following washings/treatments with CH₂Cl₂ (3 x 0.5 min), DMF (3 x 0.5 min), piperidine as indicated in General Procedures, and DMF (5 x 0.5 min). The loading calculated by Fmoc determination was 0.50 mmol/g.

### Step 2

### Fmoc-D-allo-Ile-D-allo-Thr(Val-Alloc)-D-allo-Ile-D-Val-O-TrtCl-resin.

Fmoc-D-*allo*-Ile-OH (707 mg, 2 mmol, 4 equiv), Fmoc-D-allo-Thr-OH (free hydroxy group) (683 mg, 2 mmol), 4 equiv), and Fmoc-D-*allo-*Ile-OH (707 mg, 2 mmol, 4 equiv) were added sequentially to the above obtained H-D-Val-O-TrtCl-resin using DIPCDI (310 µL, 2 mmol, 4 equiv) and HOBt (307 mg, 2 mmol, 4 equiv) in DMF (2.5 mL). In all cases, after 90 min of coupling, the ninhydrin test was negative. Removal of Fmoc group and washings were carried out as described in General Procedures. Alloc-Val-OH (502 mg, 2.5 mmol, 5 equiv) was coupled with DIPCDI (387 mg, 2.5 mmol, 5 equiv) in the presence of DMAP (30.6 mg, 0.25 mmol, 0.5 equiv) and DIPEA (88 µL, 0.5 mmol, 1 equiv) for 45 min. This coupling was repeated in the same conditions twice. An aliqout of the peptidyl-resin was treated with TFA and the HPLC (t*_{R}* 7.8 min, conditions S, column D) of the crude obtained after evaporation showed a purity of > 98%. ESMS, calcd for C₄₅ H₆₃ N₅ O₁₁, 849.45.

Found: *m*/*z* 850.1 [M+H]⁺.

### Step 3

### Fmoc-D-Val-D-Pro-Orn(Boc)-D-allo-Ile-D-allo-Thr(Val-Alloc)-D-allo-Ile-D-Val-O-TrtCl-resin.

The Fmoc group was removed and Fmoc-Orn(Boc)-OH (912 mg, 2 mmol, 4 equiv), Fmoc-D-Pro-OH (843 mg, 2.5 mmol, 5 equiv), and Fmoc-D-Val-OH (255 mg, 2.5 mmol, 5 equiv) were sequentially added to the above peptidyl-resin (Step 2) using DIPCDI (310 µL, for 2.0 mmol and 4 equiv; and 388 µL, for 2.5 mmol, 5 equiv) and HOBt (307 mg, for 2.0 mmol and 4 equiv; and 395 mg, 2.5 mmol. 5 equiv) for 90 min. Ninhydrin test after incorporation of Orn and D-Pro was negative. The chloranil after incorporation of D-Val was slightly positive and therefore a recoupling of this residue was carried out with Fmoc-D-Val-OH (678 mg, 2.0 mmol, 4 equiv), DIPCDI (310 µL, 2.0 mmol), 4 equiv) and HOBt (307 mg, 2.0 mmol, 4 equiv) for 90 min. An aliquot of the peptidyl-resin was treated with TFA and the HPLC (t*_{R}* 10.1 1 min, conditions S, column D) of the crude obtained after evaporation showed a purity of > 98 %. MALDI-TOF-MS, calcd for C₅₅ H₉₇ N₉ O₁₆, 1,259.71. Found: *m*/*z* 1,282.16 [M+Na]⁺.

### Step 4

### (4,S)-MeHex-D-Val-Thr(tBu)-Val-D-Val-D-Pro-Orn(Boc)-D-allo-Ile-D-allo-Thr(Val-Alloc)-D-allo-Ile-D-Val-O-TrtCl-resin

The Fmoc group was removed and Fmoc-Val-OH (678 mg, 2 mmol, 4 equiv), Fmoc-Thr(*t*Bu)-OH (992 mg, 2.5 mmol), 5 equiv), Fmoc-D-Val-OH (678 mg, 2 mmol, 4 equiv), and (4*S*)-MeHex-OH (195 mg, 1.5 mmol, 3 equiv) were sequentially added to the above peptidyl-resin (Step 3) using DIPCDI (233 µL, for 1.5 mmol and 3 equiv; 310 µL, for 2 mmol and 4 equiv; and 388 µL, for 2.5 mmol, 5 equiv) and HOBt (230 mg, for 1.5 mmol and 3 equiv; 307 mg, for 2 mmol and 4 equiv; and 395 mg, 2.5 mmol. 5 equiv) for 90 min. In all cases, after 90 min of coupling, the ninhydrin test was negative. Removal of Fmoc group and washings were carried out as described in General Procedures.

### Step 5

### (4S)-MeHex-D-Val-Thr(tBu)-Val-D-Val-D-Pro-Orn(Boc)-D-allo-Ile-D-allo-Thr(Val-Z-Dhb-Phe-Alloc)-D-allo-Ile-D-Val-O-TrtCl-resin.

Alloc group was removed with Pd(PPh₃)₄ (58 mg, 0.05 mmol, 0.1 equiv) in the presence of PhSiH₃ (617 µL, 5 mmol, 10 equiv) under atmosphere of Ar and Alloc-Phe-Z-Dhb-OH (666 mg, 2 mmol, 4 equiv) and HOAt (273 mg, 2 mmol), 4 equiv) were dissolved in DMF (1.25 mL) and added to peptidyl-resin, then DIPCDI (310 µL, 2 mmol, 4 equiv) was added and the mixture stirred for 5 h, where the ninhydrin test was negative. After washings with DMF and CH₂Cl₂, an aliquot of the peptidyl-resin was treated with TFA-H₂O (1:99) for 1 min and the product was characterized by MALDI-TOF-MS, calcd for C₈₈ H₁₄₆ N₁₄ O₂₁, 1,735.08. Found: *m*/*z* 1,758.67 [M+Na]⁺, 1,774.62 [M+K]⁺.

### Step 6

### (4S)-MeHex-D-Val-Thr(tBu)-Val-D-Val-D-Pro-Om(Boc)-D-allo-Ile-D-allo-Thr(Val-Z-Dhb-Phe-H)-D-allo-Ile-D-Val-OH.

After washings with DMF and CH₂Cl₂, the Alloc group was removed with Pd(PPh₃)₄ (58 mg, 0.05 mmol, 0.1 equiv) in the presence of PhSiH₃ (617 µL, 5 mmol, 10 equiv) under atmosphere of Ar. The protected peptide was cleaved from the resin by TFA-CH₂Cl₂ (1:99) (5 x 30 sec). Filtrate was collected on H₂O (4 mL) and the H₂O was partially removed in a rotavapor. ACN was then added to dissolve solid that appeared during the H₂O removal, and the solution was lyophilized, to give 639 mg (387 µmol, 77% yield) of the title compound with a purity of > 95 % as checked by HPLC (Condition R, column C, t*_{R}* 10.5 min).

### Step 7

### (4S)-MeHex-D-Val-Thr-Val-D-Val-D-Pro-Orn-D-allo-Ile-cyclo(D-allo-Thr-D-allo-Ile-D-Val-Phe-Z-Dhb-Val)

The protected peptide (Step 6) (639 mg, 387 µmol) was dissolved in CH₂Cl₂ (390 mL, 1 mM), and HOBt (237 mg, 1.55 mmol) dissolved in the minimum volume of DMF to dissolve HOBt, DIPEA (203 µL, 1.16 mmol, 3 equiv), and DIPCDI (240 µL, 1.55 mmol, 4 equiv) were added. The mixture was allowed to stir for 1 h, then the course of the cyclization step was checked by HPLC. The solvent was removed by evaporation under reduced pressure. The protected cyclic peptide was dissolved in TFA-H₂O (19:1, 85 mL) and the mixture was allowed to stir for 1 h. The solvent was removed by evaporation under reduced pressure, and dioxane is added (30 mL) and the solvent is removed by evaporation under reduced pressure (the process was repeated three times), then H₂O (40 mL) was added and lyophilized. The crude product was purified by HPLC (Kromasil C₈ 5 µm, 205 x 50 mm), isocratic 44% acetonitrile (+0.05% TFA) in water (+0.05% TFA), 55 mL/h, detection at 220 nm, to give the title product (192 mg, 0.13 mmol), 26% yield, 92.3%). MALDI-TOF-MS, calcd for C₇₅ H₁₂₄ N₁₄ O₁₆, 1,476.93. Found: *m*/*z* 1,500.12 [M+Na]⁺, 1,515.97 [M+K]⁺. The ¹H-NMR (2.5 mM, 500 MHz, H₂O-D₂O (9:1) spectrum of the compound is indicated in Table II).

**Table II**

| RESIDE | N-H | Hα | Hβ | OTHER |
|---|---|---|---|---|
| (Z)-Dhb | 9.59 (s) | - | 6.63 (q, J =7.5 Hz) | 1.19 (d, γ-CH₃) |
| D-al·lo-Ile 1 | 8.82 (d, J =9.0 Hz) | 4.42 | 1.87 | 1.25, 1.09, 0.82 (γ-CH₂, γ-CH₃, δ-CH₃) |
| L-Phe | 8.75 (d, J =5.5 Hz) | 4.63 | 3.08 (m) | 7.31 (2H·Ar, t), 7.25 (3H Ar, d) |
| D-al·lo-Thr | 8.67(d, J =9.0 Hz) | 4.64 | 5.05 (m) | 1.21 (γ-CH3) |
| D-Val 3 | 8.13 (d, J =7.5 Hz) | 4.33 | 2.01 | 0.90 (2 γ-CH₃) |
| L-Orn | 8.29 (d, J =7.5 Hz) | 4.31 | 1.66 (2H) | 1.88 (γ-CH₂), 2.96 (bs, δ-CH₂), 7.65 (ε-NH₃⁺) |
| D-al·lo-Ile 2 | 7.92 (d) | 4.18 | 1.80 | 1.25, 1.09, 0.81 (γ-CH₂, γ-CH₃, δ-CH₃) |
| D-Val 5 | 8.01 (d) | 4.08 | 2.07 | 0.87 (2 γ-CH₃) |
| L-Thr | 8.19 (d, J =7.5 Hz) | 4.29 | 4.14 (m) | 1.13 (γ-CH₃) |
| D-Val 2 | 7.89 (d, J =7.5 Hz) | 4.10 | 2.11 | 0.78 (γ-OH₃) |
| L-Val 4 | 8.04 (d) | 4.10 | 2.07 | 0.90 (2 γ-CH₃) |
| L-Val 1 | 7.19 (d, J =9.0 Hz) | 4.02 | 1.52 | 0.75 (γ-CH₃), 0.65 (d, γ-CH₃) |
| D-Pro | - | 4.36 | 2.23, 1.99 (m, β-CH₂), 1.85 (m, γ-CH₂), 3.83 (1H, m, δ-CH₂), 3.64 (1H, m, δ-CH₂) | |
| 4(S)-MeHex | - | 2.26 (2H) | 1.57 (β-CH₂). 1.26, 1.10, 1.33, 0.79 (δ-CH₂, δ-CH₃, γ-CH, ε-CH₃) | |

### Example 2

Analogues described in Table III were synthesized following experimental procedures as described in Example 1, except the step indicated into the column (Step), where residue(s) (A) was replaced by other(s) (B) or removed (none).

**Table III**

| **Analogue** | **Compound** # | **Step** | **Residue Replaced (A)** | **Residue Incorporate (B)** |
|---|---|---|---|---|
| [D-Thr⁶]-KF | 2 | 2 | Fmoo-Dallo-Thr-OH 6 | Fmoc-D-Thr-OH |
| | | 4 | (4S)-MeHex 14 | 5-MeHex |
| [D-Ser⁶]-KF | 3 | 2 | Fmoc-Dallo-Thr-OH 6 | Fmoc-D-Ser-OH |
| | | 4 | (4S)-MeHex 14 | 5-MeHex |
| [Glu⁸]-KF | 4 | 3 | Fmoc-Orn(Boc)-OH 8 | Fmoc-Glu(^{t}Bu)-OH |
| | | 4 | (4*S*)-MaHex 14 | 5-MeHex |
| [Lys⁸]-KF | 5 | 3 | Fmoc-Orn(Boc)-OH 8 | Fmoc-Lys(Boc)-OH |
| | | 4 | (4*S*)-MeHex 14 | 5-MeHex |
| [Val¹²]-KF | 6 | 4 | Fmoc-Thr(^{t}Bu)-OH 12 | Fmoc-Val-OH |
| | | | (4*S*)-MeHex 14 | 5-MeHex |
| [D-Thr¹²]-KF | 7 | 4 | Fmoc-Thr(^{t}Bu)-OH 12 | Fmoc-D-Thr(^{t}Bu)-OH |
| | | | (4*S*)-MeHex 14 | 5-MeHex |
| [D-Cha¹³]-KF | 8 | 4 | Fmoc-D-Val-OH 13 | Fmoc-D-Cha-OH |
| | | | (4*S*)-MeHex 14 | 5-MeHex |
| [hCh¹¹]-KF | 9 | 4 | Fmoc-Val-OH 11 | Fmoc-hCh-OH |
| | | | (4*S*)-MeHex 14 | 5-MeHex |
| [hCh¹¹, D-Cha¹³]-KF | 10 | 4 | Fmoc-Val-OH 11 | Fmoc-hCh-OH |
| | | | Fmoc-D-Val-OH 13 | Fmoc-D-Cha-OH |
| | | | (4*S*)-MeHex 14 | 5-MeHex |
| [D-Cha⁴, D-Cha⁵ ,D- | 11 | 1 | Fmoc-D-Val-OH 4 | Fmoc-D-Cha-OH |
| | | 2 | Fmoc-D-alo-Ile-OH 5 | Fmoc-D-Cha-OH |
| | | 2 | Fmoc-D-alo-Ile-OH 7 | Fmoc-D-Cha-OH |
| | | 4 | (4*S*)-MeHex 14 | 5-MeHex |
| [D-Val⁵ ,D-Val⁷]-KF | 12 | 2 | Fmoc-D-alo-Ile-OH 5 | Fmoc-D-Val-OH |
| | | 2 | Fmoc-D-alo-Ile-OH 7 | Fmoc-D-Val-OH |
| | | 4 | (4*S*)-MeHex 14 | 5-MeHex |
| [Icos¹⁴]-KF | 13 | 4 | (4*S*)-MeHex 14 | Icosanoic acid |
| [(c/t)-4-Me-cHexa¹⁴]-KF | 14 | 4 | (4*S*)-MeHex 14 | cis/trans-2-(4-Methyl-cyclohexyl)acetic acid |
| [Und¹⁴]-KF | 15 | 4 | (4*S*)-MeHex 14 | Undecanoic acid |
| [(4*R*)-MeHex¹⁴]-KF | 16 | 4 | (4*S*)-MeHex 14 | (4*R*)-Methyl hexanoic acid |
| [(4R*S*)-MeHex¹⁴]-KF | 17 | 4 | (4*S*)-MeHex 14 | (4*RS*)-Methylhexanoic acid |
| [Oct¹⁴]-KF | 18 | 4 | (4*S*)-MeHex 14 | Octanoic acid |
| [*p*-MeBza¹⁴]-KF | 19 | 4 | (4*S*)-MeHex 14 | *p*-Methylbenzoic acid |
| [Bza¹⁴]-KF | 20 | 4 | (4*S*)-MeHex 14 | Benzoic acid |
| [*p*-CF₃Bza¹⁴]-KF | 21 | 4 | (4*S*)-MeHex 14 | *p*-Trifluoromethylibenzoic acid |
| [3,5-dFPhAc¹⁴]-KF | 22 | 4 | (4*S*)-MeHex 14 | 3,5-Dlfluorophenylacetlc acid |
| [Pipe¹⁴]-KF | 23 | 4 | (4*S*)-MeHex 14 | Piperonilic acid |
| [*p*-CF₃Clnn¹⁴]-KF | 24 | 4 | (4*S*)-MeHex 14 | *p*-Trifluoromethylcinnamic acid |
| [*p*-CF₃PhAc¹⁴]-KF | 25 | 4 | (4*S*)-MeHex 14 | *p*-Trifluoromethylphenylacet Ic acid |
| [Pfh¹⁴]-KF | 26 | 4 | (4*S*)-MeHex 14 | Perfluoroheptanolc acid |
| [6-OHep¹⁴]-KF | 27 | 4 | (4*S*)-MeHex 14 | 6-Oxoheptanoio acid |
| [6,6-dFHep¹⁴]-KF | 28 | 4 | (4*S*)-MeHex 14 | 6,6-Difluoroheptanoic acid |
| [4-GuBut¹⁴]-KF | 29 | 4 | (4*S*)-MeHex 14 | 4-([Amino(imino)methyl]ami no)butanoic acid |
| [Lys⁸, (4S)-MeHeX¹⁴]-KF | 30 | 3 | Fmoc-Drn(Boc)-OH 8 | Fmoc-Lys(Boc)-OH |
| [noVal¹¹, noThr¹², noD-Val¹³]-KF | 31 | 3 | Fmoc-Val-OH 11 | none |
| | | 4 | Fmoc-Thr(tBu)-12 | none |
| | | 4 | Fmoc-D-Val-OH 13 | none |
| | | 4 | (4*S*)-MeHex 14 | 5-MeHex |
| [noVal¹¹, nOThr¹², noD-Val¹³, Mst¹⁴]-KF | 32 | 4 | Fmoc-Val-OH 11 | none |
| | | 4 | Fmoc-Thr(tBu)-12 | none |
| | | 4 | Fmoc-D-Val-OH 13 | none |
| | | 4 | (4*S*)-MeHex 14 | Myristic (tetradecanolc) acid |
| [Gly¹³]-KF | 33 | 4 | Fmoc-D-Val-OH 13 | Fmoc-Gly-ON |
| | | | (4*S*)-MeHex 14 | 5-MeHex |
| [D-Ala¹³]-KF | 34 | 4 | Fmoc-D-Val-OH 13 | Fmoc-D-Ala-OH |
| | | | (4*S*)-MeHex 14 | 5-MeHex |
| [D-Leu¹³]-KF | 35 | 4 | Fmoc-D-Val-OH 13 | Fmoc-D-Leu-OH |
| | | | (4*S*)-MeHex 14 | 5-MeHex |
| [D-Phe¹³]-KF | 36 | 4 | Fmoc-D-Val-OH 13 | Fmoc-D-Phe-OH |
| | | | (4*S*)-MeHex 14 | 5-MeHex |
| [D-Pro¹³]-KF | 37 | 4 | Fmoc-D-Val-OH 13 | Fmoc-D-Pro-OH |
| | | | (4*S*)-MeHex 14 | 5-MeHex |
| [Val¹³]-KF | 38 | 4 | Fmoc-D-Val-OH 13 | Fmoc-Val-OH |
| | | | (4*S*)-MeHex 14 | 5-MeHex |
| [D-Glu¹³]-KF | 39 | 4 | Fmoc-D-Val-OH 13 | Fmoc-D-Glu-OH |
| | | | (4*S*)-MeHex 14 | 5-MeHex |
| [D-Gln¹³]-KF | 40 | 4 | Fmoc-D-Val-OH 13 | Fmoc-D-Gln-OH |
| | | | (4*S*)-MeHex 14 | 5-MeHex |
| [D-Thr¹³]-KF | 41 | 4 | Fmac-D-Val-OH 13 | Fmoc-D-Thr-OH |
| | | | (4*S*)-MeHex 14 | 5-MeHex |
| [Gly¹¹]-KF | 42 | 4 | Fmoc-Val-OH 11 | Fmoc-Gly-OH |
| | | | (4*S*)-MeHex 14 | 5-MeHex |
| [Phe¹¹]-KF | 43 | 4 | Fmoc-Val-OH 11 | Fmoc-Phe-OH |
| | | | (4*S*)-MeHex 14 | 5-MeHex |
| [Ala¹¹]-KF | 44 | 4 | Fmoc-Val-OH 11 | Fmoc-Ala-OH |
| | | | (4*S*)-MeHex 14 | 5-MeHex |
| [Leu¹¹]-KF | 45 | 4 | Fmoc-Val-OH 11 | Fmoc-Leu-OH |
| | | | (4*S*)-MeHex 14 | 5-MeHex |
| [D-Val¹¹]-KF | 46 | 4 | Fmoc-Val-OH 11 | Fmoc-D-Val-OH |
| | | | (4*S*)-MeHex 14 | 5-MeHex |
| [Pro¹¹]-KF | 47 | 4 | Fmoc-Val-OH 11 | Fmoc-Pro-OH |
| | | | (4*S*)-MeHex 14 | 5-MeHex |
| [Gln¹¹]-KF | 48 | 4 | Fmoc-Val-OH 11 | Fmoc-Gln-OH |
| | | | (4*S*)-MeHex 14 | 5-MeHex |
| [Orn¹¹]-KF | 49 | 4 | Fmoc-Val-OH 11 | Fmoc-Orn-OH |
| | | | (4*S*)-MeHex 14 | 5-MeHex |
| [Thr¹¹]-KF | 50 | 4 | Fmoc-Val-OH 11 | Fmoc-Thr-OH |
| | | | (4*S*)-MeHex 14 | 5-MeHex |
| [Glu¹¹]-KF | 51 | 4 | Fmoc-Val-OH 11 | Fmoc-Glu-OH |
| | | | (4*S*)-MeHex 14 | 5-MeHex |
| [Ala¹², noD-Val¹³]-KF | 52 | 4 | Fmoc-Thr-OH 12 | Fmoc-Ala-OH |
| | | | rmoc-D-Val-OH 13 | none |
| [Gly¹², noD-Val¹³]-KF | 53 | 4 | (4*S*)-MeHex 14 | 5-MeHex |
| | | | Fmoc-Thr-OH 12 | Fmoc-Gly-OH |
| [Leu¹², noD-Val¹³]-KF | 54 | 4 | Fmoc-D-Val-OH 13 | none |
| | | | (4*S*)-MeHex 14 | 5-MeHex |
| [Pro¹², noD-Val¹³]-KF | 55 | 4 | Fmoc-Thr-OH 12 | Fmoc-Pro-OH |
| | | | Fmoc-D-Val-OH 13 | none |
| [Glu¹², noD- | 56 | 4 | (4*S*)-MeHex 14 | 5-MeHex |
| | | | Fmoc-Thr-OH 12 | Fmoc-Glu-OH |
| [Orn¹², noD-Val¹³]-KF | 57 | 4 | Fmoc-Thr-OH 12 | Fmoo-Orn-OH |
| | | | Fmoc-D-Val-OH 13 | none |
| [Gln¹², noD-Val¹³]-KF | 58 | 4 | Fmoc-L-Thr-OH 12 | Fmoc-Gln-OH |
| | | | Fmoc-D-Val-OH 13 | none |
| [Pro⁸, (4S)-MeHex¹⁴]-KF | 59 | 4 | Fmoc-D-Pro-OH 9 | Fmoc-Pro-OH |
| [D-Pip⁹, (4S)-MeHex¹⁴]-KF | 60 | 4 | Fmoc-D-Pro-OH 9 | Fmoc-D-Pip-OH |
| [D-Tic⁹, (4S)-MeHex¹⁴]-KF | 61 | 4 | Fmoc-D-Pro-OH 9 | Fmoc-D-Tic-OH |
| [(5R)-Ph-Pro⁹, (4S)-MeHex¹⁴]-KF | 62 | 4 | Fmoc-D-Pro-OH 9 | Fmoc-(5R)-Ph-Pro-OH |
| [L-Val-d₈¹¹]-KF | 100 | 4 | Fmoc-L-Val-OH 11 | Fmoc-L-Val-d₈-OH |
| | | | (4S)-MeHex 14 | 5-MeHex |
| [AM¹⁴]-KF | 101 | 4 | (4S)-MeHex 14 | AM |
| [AO¹⁴]-KF | 102 | 4 | (4S)-MeHex 14 | AO |
| [[C(=N(CH₃) ₂)¹⁴]-KF | 103 | 4 | (4S)-MeHex 14 | C(=N(CH₃)₂) |
| [D-Ile⁷]-KF | 104 | 2 | Fmoc-D-allo-Ile-OH 7 | Fmoc-D-Ile-OH |
| | | 4 | (4*S*)-MeHex 14 | 5-MeHex |
| [D-Val⁷]-KF | 105 | 2 | Fmoc-D-allo-Ile-OH 7 | Fmoc-D-Val-OH |
| | | 4 | (4*S*)-MeHex 14 | 5-MeHex |
| [D-Orn⁸, L-Pro⁹]-KF | 106 | 3 | Fmoc-L-Orn-OH 8 | Fmoc-D-Orn-OH |
| | | | Fmoc-D-Pro-OH 9 | Fmoc-L-Pro-OH |
| | | 4 | (4*S*)-MeHex 14 | 5-MeHex |
| [D-Cys⁷, L-Cys¹¹]-KF | 107 | 2 | Fmoo-D-allo-Ile-OH 7 | Fmoc-D-Cys(Trt)-OH |
| | | 4 | Fmoc-L-Val-OH 11 | Fmoc-L-Cys(Trt)-OH |
| | | | (4*S*)-MeHex 14 | 5-MeHex |
| [D-Cys⁷, D-Cys¹⁰]-KF | 108 | 2 | Fmoo-D-allo-Ile-OH 7 | Fmoc-D-Cys(Trt)-OH |
| | | 3 | Fmoc-D-Val-OH 10 | Fmoc-D-Cys(Trt)-OH |
| | | 4 | (4*S*)-MeHex 14 | 5-MeHex |
| [D-homoCys⁷, D-homoCys¹⁰]-KF | 109 | 2 | Fmoc-D-allo-Ile-OH 7 | Fmoc-D-homoCys(Trt)-OH |
| | | 3 | Fmoc-D-Val-OH 10 | Fmoc-D-homoCys(Trt)-OH |
| | | 4 | (4*S*)-MeHex 14 | 5-MeHex |
| [D-Val¹]-KF | 110 | 2 | Alloc-L-Val-OH 1 | Alloc-D-Val-OH |
| [Gly¹²]-KF | 111 | 4 | Fmoc-Thr(tBu)-OH 12 | Fmoc-Gly-OH |
| | | 4 | (4*S*)-MeHex 14 | 5-MeHex |
| [Ala¹²]-KF | 112 | 4 | Fmoc-Thr(tBu)-OH 12 | Fmoc-Ala-OH |
| | | 4 | (4*S*)-MeHex 14 | 5-MeHex |
| [Leu¹²]-KF | 113 | 4 | Fmoc-Thr(tBu)-OH 12 | Fmoc-Leu-OH |
| | | 4 | (4*S*)-MeHex 14 | 5-MeHex |
| [Phe¹²]-KF | 114 | 4 | Fmoc-Thr(tBu)-OH 12 | Fmoc-Phe-OH |
| | | 4 | (4*S*)-MeHex 14 | 5-MeHex |
| [Glu¹²]-KF | 115 | 4 | Fmoc-Thr(tBu)-OH 12 | Fmoc-Glu-OH |
| | | 4 | (4*S*)-MeHex 14 | 5-MeHex |
| [Orn¹²]-KF | 116 | 4 | Fmoc-Thr(tBu)-OH 12 | Fmoc-Orn-OH |
| | | 4 | (4*S*)-MeHex 14 | 5-MeHex |
| [Pro¹²]-KF | 117 | 4 | Fmoc-Thr(tBu)-OH 12 | Fmoc-Pro-OH |
| | | 4 | (4*S*)-MeHex 14 | 5-MeHex |
| [D-Orn¹³]-KF | 118 | 4 | Fmoc-D-Val-OH 13 | Fmoc-D-Om-OH |
| | | 4 | (4*S*)-MeHex 14 | 5-MeHex |
| [Gln¹²]-KF | 119 | 4 | Fmoc-Thr(tBu)-OH 12 | Fmoc-Gln-OH |
| | | 4 | (4*S*)-MeHex 14 | 5-MeHex |

### Example 3

### 5-MeHex-D-Val-Thr-Val-D-Val-D-Pro-Orn-D-allo-Ile-cyclo[D-allo-Thr-D-alo-Ile-D-Val-hCh-(Z)-Dhb-Val] (Compound 63)

Experimental procedures as decribed in Example 1, except that in step 4 (4S)-MeHex was replaced by 5-MeHex and step 5 was carried according the following experimental procedure:
Alloc group from 5-MeHex-D-Val-Thr(^{t}Bu)-Val-D-Val-D-Pro-Orn(Boc)-D-*allo*-Ile-D-alo-Thr(Val-Alloc)-D-*allo*-Ile-D-Val-O-2-Glorotrityl-Ps (250 mg, initial loading = 0.5 mmol/g resin) was removed as above with Pd(PPh₃)₄ in the presence of PhSiH₃ under atmosphere of Ar. Fmoc-Thr-OH (free hydroxy group) (213.2 mg; 0.63 mmol; 5 equiv) and Fmoc-hCha-OH (254.0 mg; 0.63 mmol, 5 equiv) were added sequentially to the above peptidyl-resin using DIPCDI (96.8 mg; 0.63 mmol; 5 equiv) and HOBt (85 mg; 0.63 mmol; 5 equiv) in DMF. After extensive washings with DMF (5 x 30 sec), the peptidyl-resin was treated with EDC·HCl (479 mg, 2,5 mmol, 20 equiv), CuCl (184 mg, 1,5 mmol, 12 equiv) in CH₂Cl₂-DMF (1:9) for 6 days. After extensively washings with DMF, CH₂Cl₂, and DMF, experimental protocols as described in example 1 was followed for obtaining the KF analogue.

### Example 4

Analogues described in Table IV were synthesized following experimental procedures as described in Example 1, except that in step 4 (4S)-MeHex was replaced by 5-MeHex; step 5 was carried as described in Example 3, but incorporating Fmoc-Phe-OH instead of Fmoc-hCh-OH, and in the step indicated into the column (Step), residue(s) (A) was replaced by other(s) (B) or removed (none). In analogues 64-66, the reaction of dehydration was not employed, because the analogues are hydrogenated.

**Table IV**

| **Analogue** | **Compound** # | **Step** | **Residue Replaced (A)** | **Residue Incorporated (B)** |
|---|---|---|---|---|
| [D,L-Ser²]-KF | 64 | 5 | Fmoc-Thr-OH 2 | Fmoc-Ser-OH |
| [Gly²]-KF | 65 | 5 | Fmoc-Thr-OH 2 | Fmoc-Gly-OH |
| [Aib²]-KF | 66 | 5 | Fmoc-Thr-OH 2 | Fmoc-Alb-OH |
| [Dha²]-KF | 67 | 5 | Fmoc-Thr-OH 2 | Fmoc-Ser-OH |
| [Trp³]-KF | 68 | 5 | Fmoc-Thr-OH 2 | Fmoc-Trp-OH |
| [D-Val¹, D-Phe³, Val⁴, aro-Ile⁵, alo-Thr⁶, alo-Ile⁷, D-Orn⁸, Pro⁹, Val¹⁰, D-Val¹¹, D-Thr¹², Val¹³] KF | 69 | 2 | Alloc-Val-OH 1 | Alloc-D-Val-OH |
| | | 5 | Fmoc-Phe-OH 3 | Fmoc-D-Phe-OH |
| | | 1 | Fmoc-D-Val-OH 4 | Fmoc-Val-OH |
| | | 2 | Fmoc-D-*allo*-Ile-OH 5 | Fmoc-*allo*-Ile-OH |
| | | 2 | Fmoc-D-*allo*-Thr-OH 6 | Fmoc-*allo*-Thr-OH |
| | | 2 | Fmoc-D-*allo*-Ile-OH 7 | Fmoc-*allo*-Ile-OH |
| | | 3 | Fmoc-Orn(Boo)-OH 8 | Fmoc-D-Orn(Boc)-OH |
| | | 3 | Fmoc-D-Pro-OH 9 | Fmoc-Pro-OH |
| | | 3 | Fmoc-D-Val-OH 10 | Fmoc-Val-OH |
| | | 4 | Fmoc-Val-OH 11 | Fmoc-D-Val-OH |
| | | 4 | Fmoc-Thr(tBu)-OH 12 | Fmoc-D-Thr(tBu-OH |
| | | 4 | Fmoc-D-Val-OH 13 | Fmoc-Val-OH |

### Example 5

Analogues described in Table V were synthesized following experimental procedures as described in Example 1, except that step 5 was carried as described in Example 3, and in the step indicated into the column (Step) residue(s) (A) have been replaced by other(s) (B). Furthermore, before solid-phase dehydratation, the Fmoc group was removed as described in General Procedures, and then the amino group was protected in form of Alloc by reaction with Alloc-OSu (5 equiv) in the presence of DIPEA (5 equiv) using DMF as a solvent (2 hours).

**Table V**

| **Analogue** | **Compound #** | Step | **Residue Replaced (A)** | **Residue Incorporated (B)** |
|---|---|---|---|---|
| [Phe(3,4-Cl₂)³, (4S)-MeHex¹⁴]-KF | 70 | 5 | Fmoc-hCh-OH 3 | Fmoc-Phe(3,4-Cl₂)-OH |
| [Phe(F₅)³, (4S)-MeHex¹⁴]-KF | 71 | 5 | Fmoc-hCh-OH 3 | Fmoc-Phe(F₅)-OH |
| [Phe(4-I)³, (4S)-MeHex¹⁴]-KF | 72 | 5 | Fmoc-hCh-OH 3 | Fmoc-Phe(4-I)-OH |
| [Phe(4-NO₂)³, (4S)-MeHex¹⁴]-KF | 73 | 5 | Fmoc-hCh-OH 3 | Fmoc-Phe(4-NO₂)-OH |
| [Phe(4-F)³, (4S)-MeHex¹⁴]-KF | 74 | 5 | Fmoc-hCh-OH 3 | Fmoc-Phe(4-F)-OH |
| [Tyr(Me)³, (4S)-MeHex¹⁴]-KF | 75 | 5 | Fmoc-hCh-OH 3 | Fmoc-Tyr(Me)-OH |
| [Thi³, (4S)-MeHex¹⁴]-KF | 76 | 5 | Fmoc-hCh-OH 3 | Fmoc-Thi-OH |
| [Tic³, (4S)-MeHex¹⁴]-KF | 77 | 5 | Fmoc-hCh-OH 3 | Fmoc-Tic-OH |
| [Tyr³, (4S)-MeHex¹⁴]-KF | 78 | 5 | Fmoc-hCh-OH 3 | Fmoc-Tyr(tBu)-OH |
| [Oic³, (4S)-MeHex¹⁴]-KF | 79 | 5 | Fmoc-hCh-OH 3 | Pmoc-Olc-OH |
| [*N*MePhe³, (4S)-MeHex¹⁴]-KF | 80 | 5 | Fmoc-hCh-OH 3 | Fmoc-*N*Me-Phe-OH |
| [Phe(2-Cl)³]-KF | 81 | 4 | (4S)-MeHex 14 | 5-MeHex |
| | | 5 | Fmoc-hCh-OH 3 | Fmoc-Phe(2-Cl)-OM |
| [Phe(S-Cl)³]-KF | 82 | 4 | (4*S*)-MeHex 14 | 5-MeHex |
| | | 5 | Fmoc-hCh-OH 3 | Fmoc-Phe(3-Cl)-OH |
| [Phe(4-Cl)³]-KF | 83 | 4 | (4*S*)-MeHex 14 | 5-MeHex |
| | | 5 | Fmoc-hCh-OH 3 | Fmoc-Phe(4-Cl)-OH |
| [Phe(3,4-F₂)³]-KF | 84 | 4 | (4*S*)-MeHex 14 | 5-MeHex |
| | | 5 | Fmoc-hCh-OH 3 | Fmoc-Phe(3,4-F₂)-OH |
| [Nal³]-KF | 85 | 4 | (4*S*)-MeWex 14 | 5-MeHex |
| | | 5 | Fmoc-hCh-OH 3 | Fmoc-Nal-OH |
| [Bip³]-KF | 86 | 4 | (4*S*)-MeHex 14 | 5-MeHex |
| | | 5 | Fmoc-hCh-OH 3 | Fmoc-Bip-OH |
| [Phg³]-KF | 87 | 4 | (4*S*)-MeHex 14 | 5-MeHex |
| | | 5 | Fmoc-hCh-OH 3 | Fmoc-Phg-OH |
| [Phe(3,4-Cl₂)³, p-CF₃Cinn¹⁴]-KF | 88 | 4 | (4*S*)-MeHex 14 | p-CF₃Clnn-OH |
| | | 5 | Fmoc-hCh-OH 3 | Fmoc-Phe(3,4-Cl₂)-OH |

### Example 6

### [N(Me)₂N'(Me)₂-Arg⁸]-Kahaialide F (Compound 89)

DIPEA (1.73 µL; 10.17 µmol) and HATU (3.86 mg; 10.15 µmol) were added to Kahalalide F (10.0 mg; 6.76 µmol) dissolved in DMF (5 ml). The reaction was followed by HPLC and after 4 h, the DMF was removed under reduced pressure and the residue was dissolved in CH₃CN-H₂O-AcOH (4.5:4.5:1, 20 ml), lyophilized, and purified by semipreparative HPLC to give the title analogue (4.5 mg, 40%).

### Example 7

### [N(Me,Ph),N'(Me)₂-Arg⁸]-Kahalalide F (Compound 90)

Experimental procedures as described in example 6, but HAPyU (4.87 mg; 10.15 µmol) was used instead of HATU: 1.63 mg, 12%.

### Example 8

### [N(CH₂)₄,N'(Me)₂-Arg⁸]-Kahalalide F (Compound 91)

Experimental procedures as described in example 6, but M₂A (4.12 mg; 10.14 µmol) was used instead of HATU: 5.2 mg, 46%.

### Example 9

### [N(CH₂)₄,N'(CH₂)₄-Arg⁸]-Kahalalide F (Compound 92a) and [N⁶(CHN(CH₂)₄,N'(CH₂)₄)-Orn⁸]-Kahalalide F (Compound 92b)

Experimental procedures as described in example 6, but BTFFH (containing an impurity of aprox. 50% of 1,1'-(fluoromethylene)dipyrrolidine) (3.15 mg; 10.16 µmol) was used instead of HATU. Two products, 92a and 92b, were obtained and it was not possible to separate them (4.0 mg of the mixture in a proportion 1:1, 35.6%).

HPLC (Cond. A, column A), t_{R}: 20.8 min (92b) 45% and t_{R}: 20.9 min (43a) 46%. EM (MALDI-TOF, m/z): (92a) calcd 1,627,05; found 1,630.8 [M+H]⁺; 1,652.6 [M+Na]⁺. (43b) calcd 1,629.06; found 1,632.7 [M+H]⁺; 1,670.6 [M+K]⁺.

### Example 10

### [N^{ε}(Me)₃-Lys⁸, (4S)-MeHex¹⁴]-KF (Compound 93)

DIPEA (10 µL, 58.8 µmol) and MeI (6µL, 0.100 mmol) were added to [Lys⁸, (4S)-MeHex¹⁴]-Kahalalide F (Compound 30) (5.0 mg; 3.35 µmol) dissolved in DMF/DCM (1:1, 5 ml). The reaction was followed by HPLC and after 12 h, the solvents were removed under reduced pressure and the residue was dissolved in CH₃CN-H₂O-AcOH (4,5:4.5:1, 20 ml), lyophilized, and purified by semipreparative HPLC to give the title analogue (2.2 mg, 44%).

### Example 11

### [Thr(OTfa)¹², 4(S)MeHex¹⁴]-Kahalalide F (Compound 94)

[4(S)MeHex¹⁴]-Kahalalide F (Compound 1; 10.0 mg; 6.7 µmol) was dissolved in TFA-DCM (1:1, 20 mL) and allowed to stir for 3 days at room temperature. Then, the solvent was removed under reduced pressure and the residue was dissolved in H₂O-CH₃CN (1:9) and purified immediately minimizing the time in that the sample was dissolved in H₂O. The fractions corresponding to the title analogue were collected in a round bottom flask submerged in liquid N₂ and lyophilized (2.5 mg; 25 %).

### Example 12

### [Orn(N⁵Tfa)⁸,Thr(OTfa)¹², 4(S)MeHex¹⁴]-Kahalalide F (Compound 95)

[(4S)-MeHex¹⁴]-KF (Compound 1; 10 mg; 6.7 µmol) was dissolved in DCM (8 ml); TFAA (18.9 µL; 134 µmol) and DIPEA (22.8 µL; 134 µmol) were added and allowed to react for 12 h. Then, the solvent was removed under reduced pressure, redissolved in H₂O-CH₃CN (1:1), and purified immediately (2.0 mg, 20 %).

### Example 13

### [Orn(N⁵Tfa)⁸, 4(S)MeHex¹⁴]-Kahalalide F (Compound 96)

Experimental procedures as described in example 12, but before purification the analogue was dissolved in H₂O-CH₃CN (1:1), left in solution by 2 h, and purified (4,3 mg, 43 %).

### Example 14

### [Thr(OTfa)¹², Lit(OTfa)¹⁴]-Kahalalide F (Compound 97)

Experimental procedures as described in Example 1, except that in the step 4 (4S)-MeHex was replaced by Lit-OH and in the step 7, the cyclic peptide was dissolved TFA-DCM (1:1, 20 ml) and was allowed to stir for 3 days at room temperature. After this time, the solvent was removed under reduced pressure from an aliquot (10%) and the solid residue was dissolved in H₂O-CH₃CN (1:9) and purified immediately minimizing the time in that the sample was dissolved in H₂O. The fractions corresponding to the title analogue were collected in a round bottom flask submerged in liquid N₂ and lyophilized (5,6 mg; 6 % yield from starting resin).

### Example 15

### [no5-MeHex¹⁴-N-(Hep)₂-D-Val¹³]-Kahalalide F (Compound 98)

Experimental procedures as described in Example 1, except that in the step 4, heptanaldehyde (60.65 µL; 0.75 mmol; 5 equiv) dissolved in DMF-AcOH (99:1; 2 mL) was added to H-D-Val-Thr(^{t}Bu)-Val-D-Val-D-Pro-Orn(Boc)-D-*allo*-Ile-D-alo-Thr(Val-Alloc)-D-*allo*-Ile-D-Val-O-2-Clorotrityl-Ps (300 mg; initial loading =0.5 mmol/g resin) and after 5 min, NaBH₃CN (28.28 mg; 0.45 mmol; 3 equiv) dissolved in DMF-AcOH (99:1; 1 mL) was added, and the mixture was allowed to react for 2 h. That treatment was repeated two more times monitoring the reaction by HPLC.

### Example 16

### [Orn(Biot)⁸]-Kahalalide F (Compound 99)

Kahalalide F (150.0 mg; 94.3 µmol), d-biotin (37.0 mg, 151.5 µmol) and HATU (114.0 mg, 299.8 µmol) were dissolved in DCM anhydrous (6.0 ml) under Ar atmosphere and NMM (58 µl, 524.0 µmol) was added. The mixture was allowed to stir for 20 hours. Then, the solvent was removed under reduced pressure and the residue was dissolved in MeOH and purified. The fractions corresponding to the title analogue were lyophilized (74.0 mg; 43 %).

### Characterization of the Kahalalide F analogues

Characterization is showed in Table VI and VII. EM in Table VI corresponds to MALDI-TOF and the exact mass was calculated by Chemwind 6.0, and EM in Table VII corresponds to APCI in positive mode.

**Table VI**

| **Analogue** | **Compou nd #** | **Chromatography** | | **t_{R} (min)** | **Exact mass** | **Found** |
|---|---|---|---|---|---|---|
| | | Cond. | Colum | | | |
| [(4S)-MeHeX¹⁴]-KF | 1 | N | C | 10.22 | 1,476.93 | 1,500.1/1,516.0 |
| [D-Thr⁶]-KF | 2 | F | C | 7.75 | 1,476.90 | 1,477.6/1,499.511, 515.4 |
| [D-Ser⁶]-KF | 3 | F | C | 7.26 | 1,462.92 | 1,463.4/1,485.4/1, 501.4 |
| [Glu⁸]-KF | 4 | A | A | 19.30 | 1,491.90 | 1,514.4/1,530.4 |
| [Lys⁸]-KF | 5 | A | A | 18.00 | 1,490.95 | 1,491.8/1,513.7/1, 529.6 |
| [Val¹²]-KF | 6 | I | C | 12.48 | 1,474.95 | 1,496.8/1,513.8 |
| [D-Thr¹²]-KF | 7 | B | C | 10.21 | 1,476.93 | 1,476.6/1,496.6/1, 514.5 |
| [D-Cha¹³]-KF | 8 | I | C | 10.28 | 1,530.98 | 1,532.0/1,553.0/1, 569.0 |
| [hCh¹¹]-KF | 9 | J | C | 11.79 | 1,544.99 | 1,544.6/1,566.3/1, 582.3 |
| [hCh¹¹, D-Cha¹³]-KF | 10 | K | C | 9.77 | 1,599.04 | 1,599.2/1,621.3/1, 637.3 |
| [D-Cha⁴, D-Cha⁵ D-Cha⁷]-KF | 11 | M | E | 6.44 | 1,611.04 | 1,612.1/1,633.1/1, 649.1 |
| [D-Val⁵, D-Val⁷]-KF | 12 | B | A | 8.56 | 1,448.90 | 1,448.5/1,470.5/1, 486.4 |
| [loos¹⁴]-KF | 13 | A | B | 23.40 | 1,559.14 | 1,662.8/1,683.7/1, 699.7 |
| [(c/t)-4-Me-cHexa¹⁴]-KF | 14 | A | A | 18.80 | 1,488.93 | 1,490.9/1,513.9/1, 528.9 |
| [Und¹⁴]-KF | 15 | A | A | 23.60 | 1,532.99 | 1,535,5/1,556.5/1, 572.3 |
| [(4R)-MsHex¹⁴]-KF | 16 | J | C | 7.30 | 1,478.93 | 1,477.8/1,499.8/1, 515.7 |
| [(4RS)-MeHex¹⁴]-KF | 17 | J | C | 7.29 | 1,476.93 | 1,478.5/1,500.5 |
| [Oct¹⁴]-KF | 16 | A | A | 12.11 | 1,490.95 | 1,491.4/1,512.4/1, 528.3 |
| [p-MeBza¹⁴]-KF | 19 | A | A | 16.70 | 1,482.88 | 1,484.6/1,506.6/1, 522.6 |
| [Bza¹⁴]-KF | 20 | A | A | 17.10 | 1,468.87 | 1.470.5/1,492.5/1. 508.5 |
| [p-CF₃Bza¹⁴]-KF | 21 | A | A | 19.90 | 1,636.86 | 1,538.1/1,560.0/1, 576.0 |
| [3,5-dFPhAc¹⁴]-KF | 22 | A | A | 17.30 | 1,518.87 | 1,520.6/1,542.6/1, 558.6 |
| [Pipe¹⁴]-KF | 23 | A | A | 17.20 | 1,512.86 | 1,514.2/1,537.2/1, 552.1 |
| [p-CF₃Cinn¹⁴]-KF | 24 | AE | A | 8.17 | 1,562.87 | 1,564.3/1,586.2/1, 602.2 |
| [p-CF₃PhAc¹⁴]-KF | 25 | A | A | 16.70 | 1,550.87 | 1,551.4/1,574.4/1, 590.4 |
| [Pfh¹⁴]-KF | 26 | A | A | 20.00 | 1,710.81 | 1,712.3/1,734.3/1, 750.3 |
| 16-OHep¹⁴]-KF | 27 | A | A | 13.80 | 1,490.91 | 1,492.6/1,514.6/1, 531.6 |
| [6,6-dFHep¹⁴]-KF | 28 | N | A | 9.52 | 1,512.91 | 1,514.5/1,636.4/1, 552.4 |
| [4-GuBut¹⁴]-KF | 29 | A | A | 12.70 | 1,491.90 | 1,493.3/1,516.3/1, 532.3 |
| [Lys⁸,(4S)-MeHex¹⁴]-KF | 30 | Q | C | 9.04 | 1,490.95 | 1,492.5/1,513.6/1, 529.5 |
| [noVal¹¹, noThr¹², noD-Val¹³]-KF | 31 | A | A | 13.70 | 1,177.75 | 1,180.9/1,202.9/1, 218.8 |
| [noVal¹¹, noThr¹², noD-Val¹³, Mst¹⁴]-KF | 32 | A | A | 20.40 | 1,275.86 | 1,279.0/1,301.0/1, 317.0 |
| [Gly¹³]KF | 33 | T | E | 12.70 | 1,434.89 | 1,436.5/1,458.0/71 8.9 |
| [D-Ala¹³]-KF | 34 | T | E | 13.50 | 1,448.90 | 1,450.6/1,472.0/72 5.7 |
| [D-Leu¹³]-KF | 35 | T | E | 15.70 | 1,490.95 | 1,492.8/1,514.9/74 6.8 |
| [D-Phe¹³]-KF | 36 | T | E | 15.70 | 1,524.93 | 1,526.5/1,548.9/76 3.9 |
| [D-Pro¹³]-KF | 37 | T | E | 14.60 | 1,474.92 | 1.476.6/1.498.1/73 9.0 |
| [Val¹³]-KF | 38 | T | E | 14.70 | 1,476.93 | 1,478.5/1,600.9/73 9.9 |
| [D-Glu¹³]-KF | 39 | T | E | 12.90 | 1,506.91 | 1,508.8/754.9 |
| [D-Gln¹³]-KF | 40 | T | E | 12.10 | 1,505.92 | 1,506.6/1,528.6 |
| [D-Thr¹³]-KF | 41 | AD | E | 18.50 | 1,478.91 | 1,480,6/1,502,b/74 1.0 |
| [Gly¹¹]-KF | 42 | T | E | 12.70 | 1,434.89 | 1,436.4/1,458.9 |
| [Phe¹¹]-KF | 43 | W | E | 39.70 | 1,524.93 | 1,526,6/1,547.6 |
| [Ala¹¹]-KF | 44 | T | E | 13.50 | 1,448.90 | 1,449.6/1,471.6 |
| [Leu¹¹]-KF | 45 | U | E | 25.70 | 1,490.95 | 1,491.7/1,513.6 |
| [D-Val¹¹]-KF | 46 | V | E | 26.50 | 1,476.93 | 1,477.6/1,499.6 |
| [Pro¹¹]-KF | 47 | X | E | 22.20 | 1,474.92 | 1,476.6/1,497.6 |
| [Gln¹¹]-KF | 48 | AC | E | 26.50 | 1,505.92 | 1,507.0/1,528.9/75 4.4 |
| [Orn¹¹]-KF | 49 | Y | E | 35.70 | 1,491.94 | 1,493.4/1,515.1/74 7.5 |
| [Thr¹¹]-KF | 50 | AB | E | 46.20 | 1,478.91 | 1,480.4/1,801.7/74 0.9 |
| [Glu¹¹]-KF | 51 | Z | E | 37.30 | 1,606.91 | 1,50B.5/754.9 |
| [Ala¹², noD-Val¹³]-KF | 52 | W | E | 37.00 | 1,347.85 | 1,348.6/1,370.6 |
| [Gly¹², noD-Val¹³]-KF | 53 | W | E | 36.60 | 1,333.84 | 1,335.4/1,357.1 |
| [Leu¹², noD-Val¹³]-KF | 54 | W | E | 40.80 | 1,389.90 | 1,391.5/1,413.2 |
| [Pro¹², noD-Val¹³]-KF | 55 | W | E | 39.50 | 1,373.87 | 1,375.411,397.1 |
| [Glu¹², noD-Val¹³]-KF | 56 | W | E | 36.10 | 1,405.86 | 1,406.6/1,428.6 |
| [Orn¹², noD-Val¹³]-KF | 57 | W | E | 31.30 | 1,390.90 | 1,392.8/1,414.1/69 7.1 |
| [Gln¹², noD-Val¹³]-KF | 58 | W | E | 35.20 | 1,404.67 | 1,406.0/1,428.0 |
| [Pro⁹, (4S)-MeHex¹⁴]-KF | 59 | R | C | 7.68 | 1,476.83 | 1,477.3/1,499.3/1, 515.2 |
| [D-Pip⁹, (4S)-MeHex¹⁴]-KF | 60 | R | C | 7.45 | 1,490.95 | 1,492.2/1,514.2/1, 530.2 |
| [D-Tic⁹, (4S)-MeHex¹⁴]-KF | 61 | R | C | 8.17 | 1,538.95 | 1,540.1/1,562.1 |
| [(5R)-Ph-Pro⁹, (4S)-MeHex¹⁴]-KF | 62 | R | C | 8.15 | 1,552.96 | 1,554.2/1,576.2 |
| [hCh³]-KF | 63 | G | C | 13.20 | 1,496.99 | 1,562.0/1,584.0/1, 600,0 |
| [D,L-Ser²]-KF | 64 | B | C | 11.15 | 1,480.93 | 1,481.1/1,503.1 |
| [Gly²]-KF | 65 | C | E | 4.27 | 1,450.92 | 1,450.5/1,472.5/1, 488.4 |
| [Aib²]-KF | 66 | D | E | 5.92 | 1,478.95 | 1,478.2/1,500.2/1, 516.2 |
| [Dha²]-KF | 67 | E | E | 4.43 | 1,462.92 | 1,463.3/1,485.3/1, 503.2 |
| [Trp³]-KF | 68 | B | C | 10.21 | 1,515.94 | 1,516.8/1,538.8/1, 554.7 |
| [D-Val¹, D-Phe³, Val⁴, allo-lle⁵, allo-Thr⁶, allo-lle⁷, D-Om⁸, Pro⁹, Val¹⁰, D-Val¹¹, D-Thr¹², Val¹³] KF | 69 | F | C | 7.30 | 1,476.93 | 1,499.3/1,515.3 |
| [Phe(3,4-Cl₂)³, (4S)-MeHex¹⁴]-KF | 70 | R | C | 8.52 | 1,544.85 | 1,545.7/1,567.6/1, 583.6 |
| [Phe(F₅)³, (4S)-MeHex¹⁴]-KF | 71 | R' | C | 8.48 | 1,568.88 | 1,567.6/1,589.6/1, 605.6 |
| [Phe(4-1)³, (4S)-MeHex¹⁴]-KF | 72 | R | C | 8.32 | 1,602.86 | 1,503,6/1,625.6/1, 641.6 |
| [Phe(4-NO₂)³, (4S)-MeHex¹⁴]-KF | 73 | R | C | 7.48 | 1,521.92 | 1,528.8/1,545.8 |
| [Phe(4-F)³, (4S)-MeHex¹⁴]-KF | 74 | R | C | 7.53 | 1,494.92 | 1,495.8/1,517.9/1, 533.9 |
| [Tyr(Me)³, (4S)-MeHex¹⁴]-KF | 75 | R | C | 7.36 | 1,506.94 | 1,508.4/1,530.4/1, 546.4 |
| [Thi³, (4S)-MeHex¹⁴]-KF | 76 | R | C | 7.40 | 1,482.89 | 1,482.9/1,504.9/1, 520.4 |
| [Tic³, (4S)-MeHex¹⁴]-KF | 77 | R | C | 7.85 | 1,488.93 | 1,489.4/1,511.4/1, 527.4 |
| [Tyr³,(4S)-MeHex¹⁴]-KF | 78 | R | C | 6.44 | 1,492.93 | 1,493.7/1,616,7/1, 531.7 |
| [Oic³, (4S)-MeHex¹⁴]-KF | 79 | R | C | 7.99 | 1,480.96 | 1,481.4/1,503.4/1, 519.4 |
| [*N*MePhe³, (4S)-MeHex¹⁴]-KF | 80 | R | C | 8.29 | 1,490.95 | 1,491.6/1,513.5 |
| [Phe(2-Cl)³]-KF | 81 | R | C | 7.95 | 1,510.89 | 1,512.3/1,534.3/1, 550.2 |
| [Phe(3-Cl)³]-KF | 82 | R | C | 7.94 | 1,510.89 | 1,512.1/1,534.0/1, 560.0 |
| [Phe(4-Cl)³]-KF | 83 | S | C | 6.75 | 1,510.89 | 1,512.3/1,534.3/1, 550.3 |
| [Phe(3,4-F₂)³-KF | 84 | R | C | 7.70 | 1,512.91 | 1,514.3/1,536.3/1, 562.3 |
| [Nal³]-KF | 85 | R | C | 8.07 | 1,526.95 | 1,528.3/1,550.2/1, 566.2 |
| [Bip³]-KF | 86 | R | C | 8.70 | 1,552.96 | 1554.3/1676.3/159 2,3 |
| [Phg³]-KF | 87 | R | C | 7.23 | 1,462.92 | 1,464.2/1.486.2/1, 502.1 |
| [Phe(3,4-Cl₂)³, p-CF₃Cinn¹⁴]-KF | 88 | R | C | 9.00 | 1,630.79 | 1632.4 |
| [*N*(Me)₂,*N*'(Me)₂-Arg⁸]-KF | 89 | A | A | 19.60 | 1,575.02 | 1,577,3/1,599.3/1, 615.3 |
| [*N*(Me,Ph),*N*'(Me) ₂-Arg⁸]-KF | 90 | A | A | 21.10 | 1,637.03 | 1,639.4/1,661.3/1, 678.3 |
| [*N*(CH₂)₄,*N*'(Me)₂-Arg⁸]-KF | 91 | A | A | 21.00 | 1,601.03 | 1,602.7/1,623.6/1, 640.6 |
| [*N*(CH₂)₄,*N*'(CH₂)₄ -Arg⁸]-KF | 92 | A | A | 20.80 | 1,629.06 | 1,630.8/1,632,7/1, 652.6/1,670.6 |
| [N°(CHN(CH₂)₄,*N'* (CH₂)₄)-Om⁸]-KF | | | | 20.90 | 1,627.05 | |
| [N^{ε}(Me)₃-Lys⁸, (4S)-MeHex¹⁴]-KF | 93 | R | C | 7.69 | 1,534.00 | 1,534.36 |
| [Thr(OTFA)¹², (4S)-MeHex¹⁴]-KF | 94 | AF | C | 6.18 | 1,572.91 | 1,498.7/1,514.7/1, 594.6/1,612.5 |
| [Orn(N°TPA)⁸, Thr(OTFA)¹², (4S)-MeHex¹⁴]-KF | 95 | L | C | 13.88 | 1,668.90 | 1,670.0/1,677.0/1. 693.0/1,574.0/1,59 6.0 |
| [Orn(N°TFA)⁸, (4S)-MeHex¹⁴]-KF | 96 | L | C | 11.42 | 1,572.91 | 1,574.0/1,596.0/1, 612.0 |
| [Thr(OTFA)¹², Lit(OTFA)¹⁴]-KF | 97 | P | C | 14.57 | 1,915.09 | 1,916.5/1,938.6/1, 820.3/1,842.4/1,72 4.6/1,746.5/1,762. 5 |
| [no 5-MeHex¹⁴, N(Hep)₂-Val¹³]-KF | 98 | A | A | 16.97 | 1,406.85 | 1.407.8/1,429.8/1, 446.8 |
| [Orn(Blot)⁸]-Kahalalide F | 99 | T | E | 16.80 | 1,703.01 | 1,704.6/1,727.0 |
| [L-Val-d₈¹¹]-KF | 100 | A | C | 10.68 | 1,484.98 | 1,508.3/1,524.2 |
| [AM¹⁴]-KF | 101 | R | C C | 8.91 | 1,819.20 | 1,820.2/1,841.9/1, 857.9 |
| [AO¹⁴]-KF | 102 | R | C | 11.07 | 1,803.19 | 1.803.9/1,825.8/1, 841.8 |
| [C(=N(CH₃)₂)14]-KF | 103 | R | C | 4.23 | 1,462.93 | 1,464.2/1,486.2/1, 503.2 |
| [D-Ile⁷]-KF | 104 | R | C | 7.35 | 1,476.93 | 1.478.5/1,500.5/1, 517.5 |
| [D-Val⁷]-KF | 105 | R | C | 7.14 | 1,462.90 | 1,464.2/1,486.1/1, 502.1 |
| [D-Orn⁸, L-Pro⁹]-KF | 106 | R | C | 7.80 | 1,476.90 | 1,477.7/1,499.7/1, 515.7 |
| [D-Cys', L-Cys¹¹]-KF | 107 | R | C | 6.53 | 1,470.80 | 1,471.9/1,493.9 |
| [D-Cys', D-cys¹⁰]-KF | 108 | R | C | 6.48 | 1,470.80 | 1,471.9/1,493.9/1, 510.9 |
| [D-homoCys⁷, D-homoCys¹⁰]-KF | 109 | R | C | 6.96 | 1,498.80 | 1,499.9/1,521.9/1, 538.9 |
| [D-Val¹]-KF | 110 | R | C | 7.37 | 1,476.90 | 1,478.0 |

**Table VII**

| **Analogue** | **Example#** | Exact mass | **Found** |
|---|---|---|---|
| [Gly¹²]-KF | 111 | 1,432.91 | 1,434.1/1,456.0 |
| [Ala¹²]-KF | 112 | 1,446.92 | 1,447.4/1,449.4 |
| [Leu¹²]-KF | 113 | 1,488.97 | 1,490.2/1,512.3/1,629.2 |
| [Phe¹²]-KF | 114 | 1,522.95 | 1,524.1/1,546.2 |
| [Glu¹²]-KF | 115 | 1,604.93 | 1,506.2/1,528.3 |
| [Orn¹²]-KF | 116 | 1,489.96 | 1,497.1/1,513.2/1,526.1 |
| [Pro¹²]-KF | 117 | 1,472.94 | 1,474.3/1.496.0 |
| ID-Orn¹³]-KF | 118 | 1,491.94 | 1,493.4/1,515.3 |
| [Gln¹²]-KF | 119 | 1,503.94 | 1,505.2/1,527.2 |

### Example 17

### Biological activity

The bioactivity of compounds of this invention is demonstrated by the results in the following tables obtained in accordance with the methodology described as follows:
The finality of this assay is to interrupt the growth of a "*in vitro"* tumor cell culture by means a continued exhibition of the cells to the sample to be testing.

### Cell Lines

| **NAME** | **N° ATCC** | **SPECIES** | **TISSUE** | **CHARACTERISTICS** |
|---|---|---|---|---|
| A-549 | CCL-185 | human | lung | lung carcinoma "NSCL" |
| SK-MEL-28 | HTB-72 | human | melanoma | malignant melanoma |
| HT-29 | HTB-38 | human | colon | colon adenocarcinoma |
| LoVo | CCL-229 | human | colon | colon adenocarcinoma |
| LoVo-Dox | | human | colon | colon adenocarcinoma (MDR) |
| DU-145 | HTB-81 | human | prostate | prostate carcinoma, not androgen receptors |
| LNCaP | CRL-1740 | human | prostate | prostate adenocarcinoma, |
| | | | | with androgen receptors |
| SK-BR-3 | HTB-30 | human | breast | breast adenocarcinoma, |
| | | | | Her2/neu+, (pleural effusion) |
| IGROV-1 | | human | ovary | ovary adenocarcinoma |
| IGROV-ET | | human | ovary | ovary adenocarcinoma, |
| | | | | characterized as ET-743 |
| | | | | resistant cells |
| SK-OV-3 | HTB-77 | human | ovary | ovary adenocarcinoma |
| | | | | (malignant ascites) |
| HeLa | CCL-2 | human | cervix | cervix epitheloid carcinoma |
| HeLa-APL | CCL-3 | human | cervix | cervix epitheloid |
| | | | | carcinoma, characterized |
| | | | | as aplidine resistant cells |
| K-562 | CCL-243 | human | bone marrow; | chronic myelogenous |
| | | | | leukemia |
| PANC-1 | CRL-1469 | human | pancreas | pancreatic epitheloid |
| | | | | carcinoma |
| HMEC-1 | | human | endothelium | |

A colorimetric type of assay, using sulforhodamine B (SRB) reaction has been adapted for a quantitative measurement of cell growth and viability [following the technique described by Philip Skehan, et al. (1990), New colorimetric cytotoxicity assay for anticancer drug screening, J. Natal. Cancer Inst., 82:1107-1112].

This form of the assay employs 96 well cell culture microplates of 9 mm diameter (Faircloth, 1988; Mosmann, 1983). Most of the cell lines are obtained from American Type Culture Collection (ATCC) derived from different human cancer types.

Cells are maintained in RPMI 1640 10% FBS, supplemented with 0.1 g/l penicillin and 0.1 g/l streptomycin sulfate and then incubated at 37°C, 5% CO₂ and 98% humidity. For the experiments, cells were harvested from subconfluent cultures using trypsin and resuspended in fresh medium before plating.

Cells are seeded in 96 well microtiter plates, at 5 x 10³ cells per well in aliquots of 195 µL medium, and they are allowed to attach to the plate surface by growing in drug free medium for 18 hours. Afterward, samples are added in aliquots of 5 µL in a ranging from 10 to 10⁻⁸ µg/mL, dissolved in DMSO:EtOH:PBS (0.5:0.5:99). After 48 hours exposure, the antitumor effect are measured by the SRB methodology: cells are fixed by adding 50 µL of cold 50% (wt/vol) trichloroacetic acid (TCA) and incubating for 60 minutes at 4°C. Plates are washed with deionized water and dried. One hundred µL of SRB solution (0.4% wt/vol in 1% acetic acid) is added to each microtiter well and incubated for 10 minutes at room temperature. Unbound SRB is removed by washing with 1% acetic acid. Plates are air dried and bound stain is solubilized with Tris buffer. Optical densities are read on a automated spectrophotometric plate reader at a single wavelength of 490 nm.

The values for mean +/- SD of data from triplicate wells are calculated. Some parameters for cellular responses can be calculated: GI = growth inhibition, TGI = total growth inhibition (cytostatic effect) and LC = cell killing (cytotoxic effect).

Tables VIII and IX illustrate data on the biological activity of the compounds of the present invention.

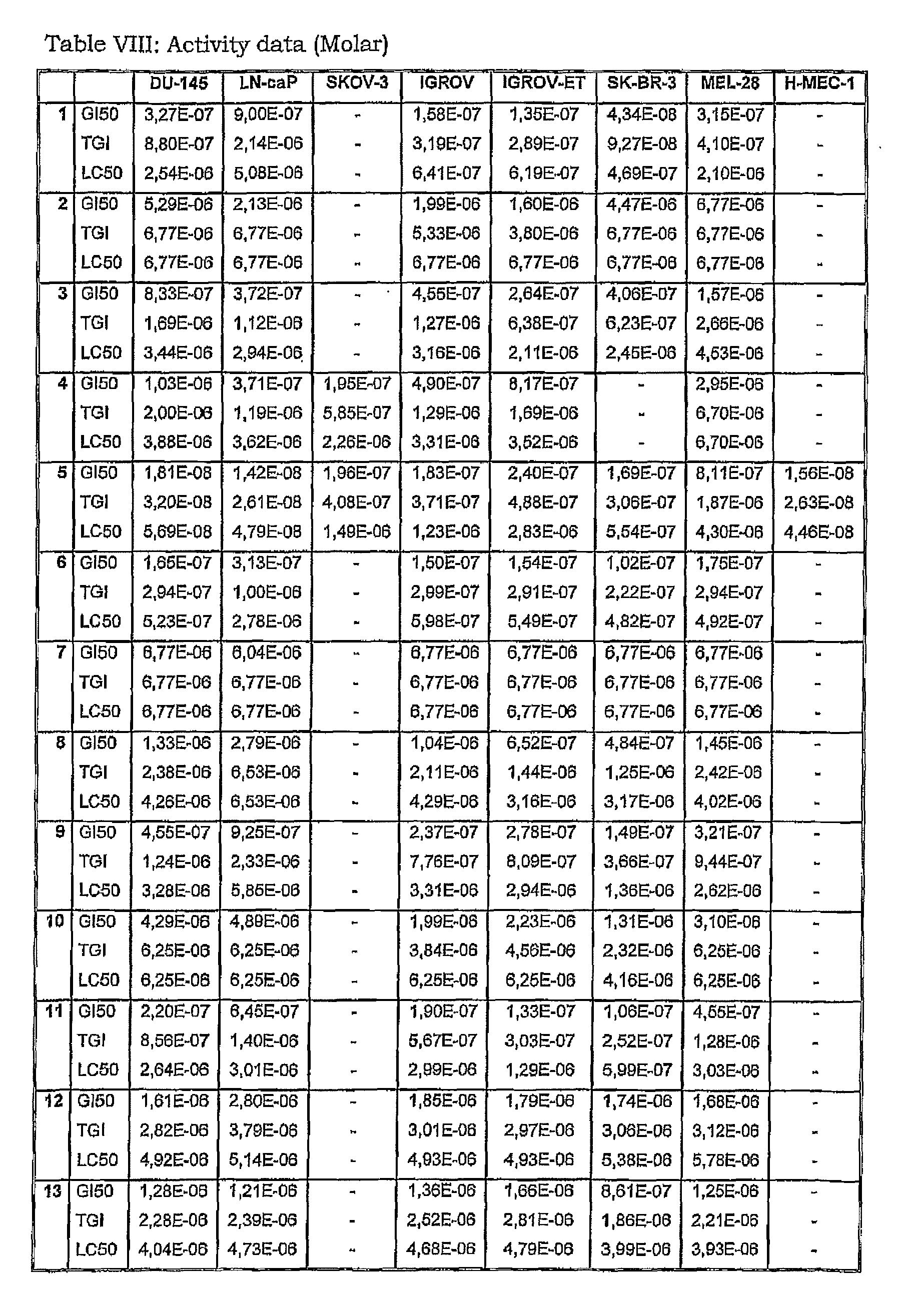

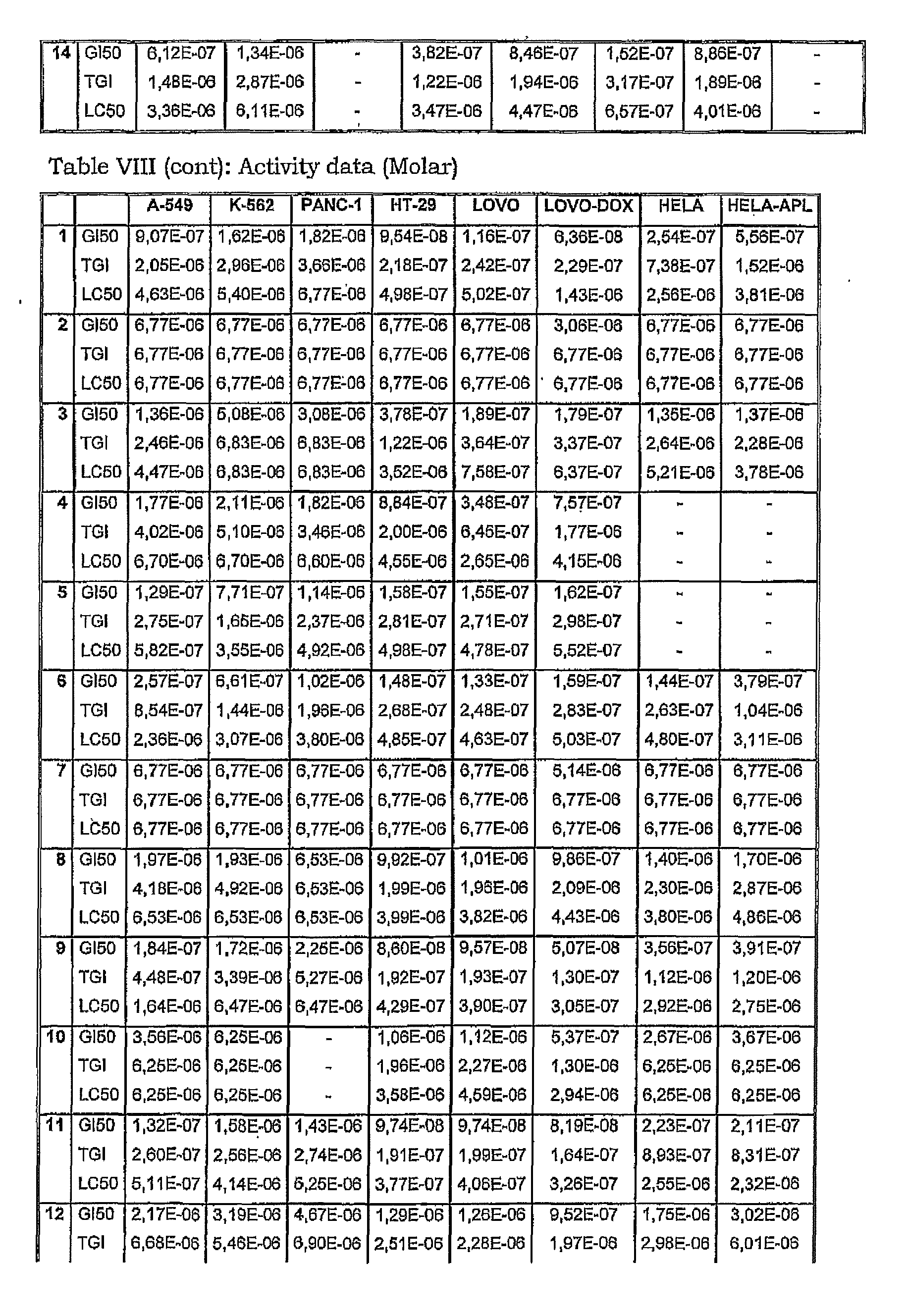

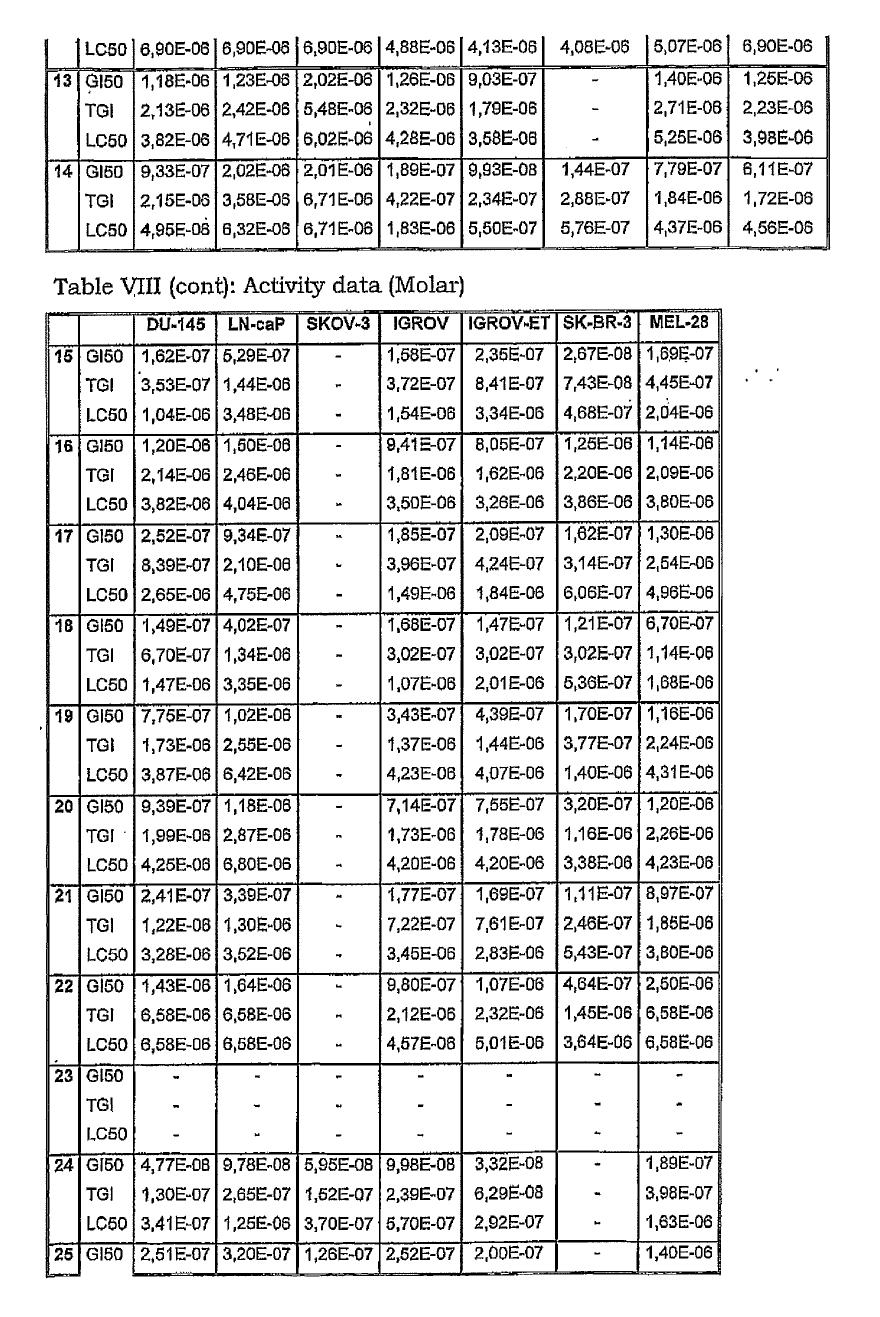

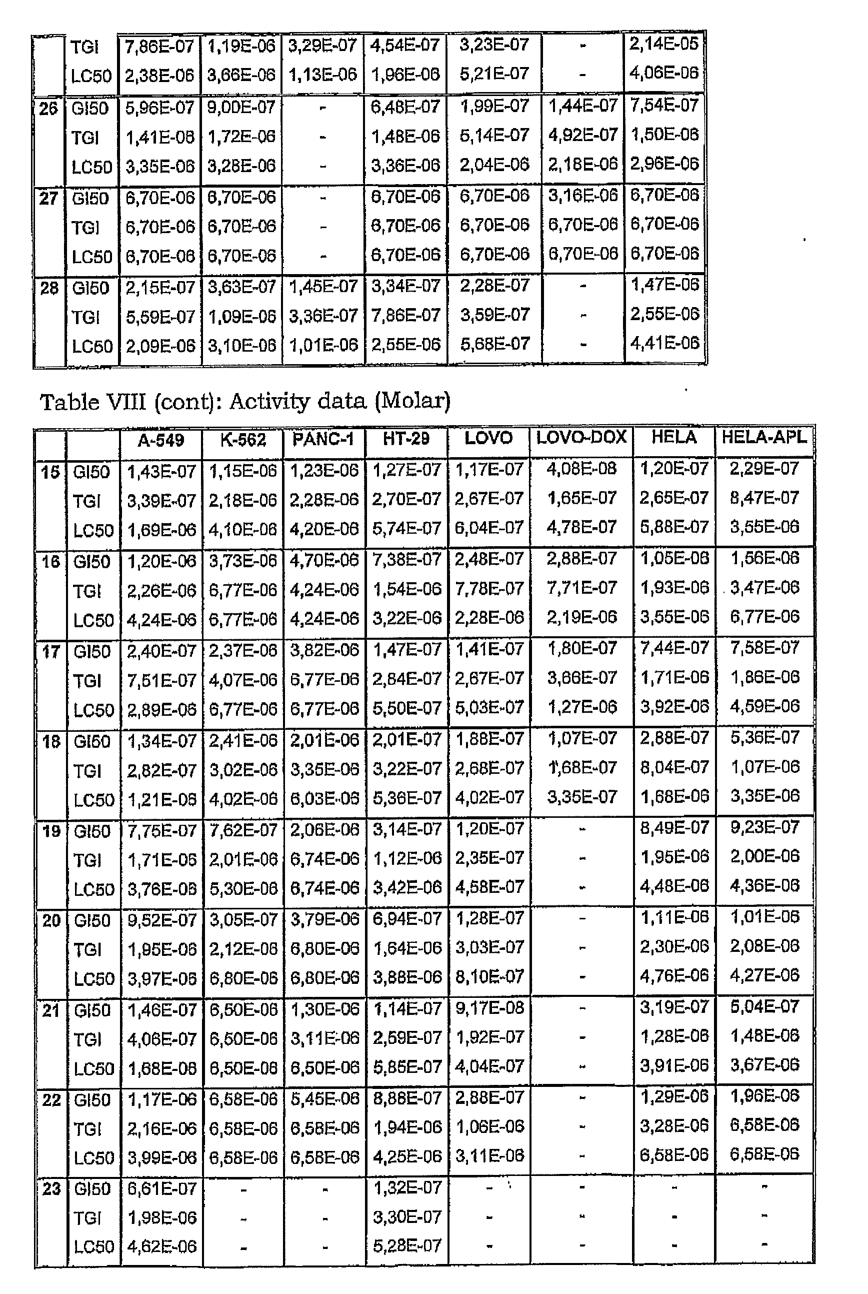

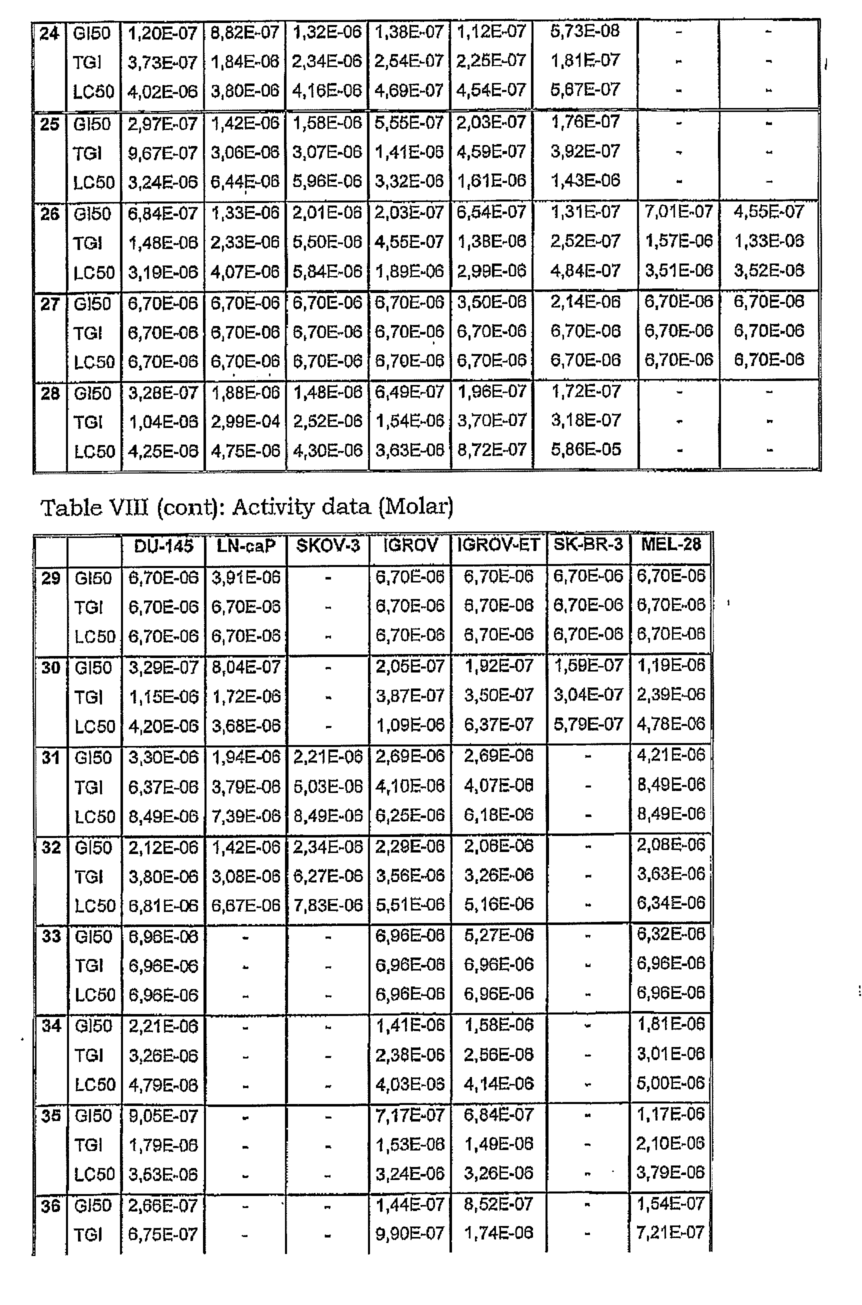

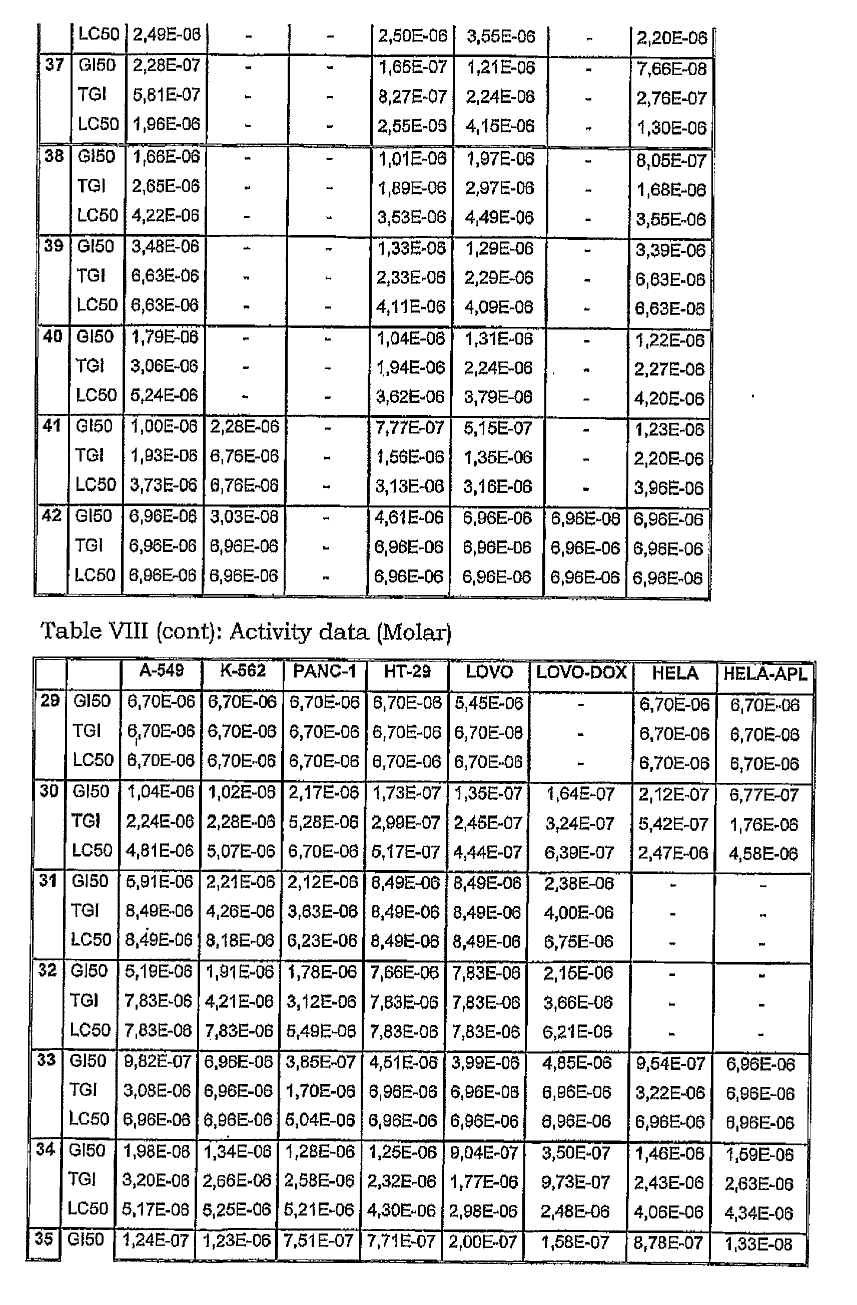

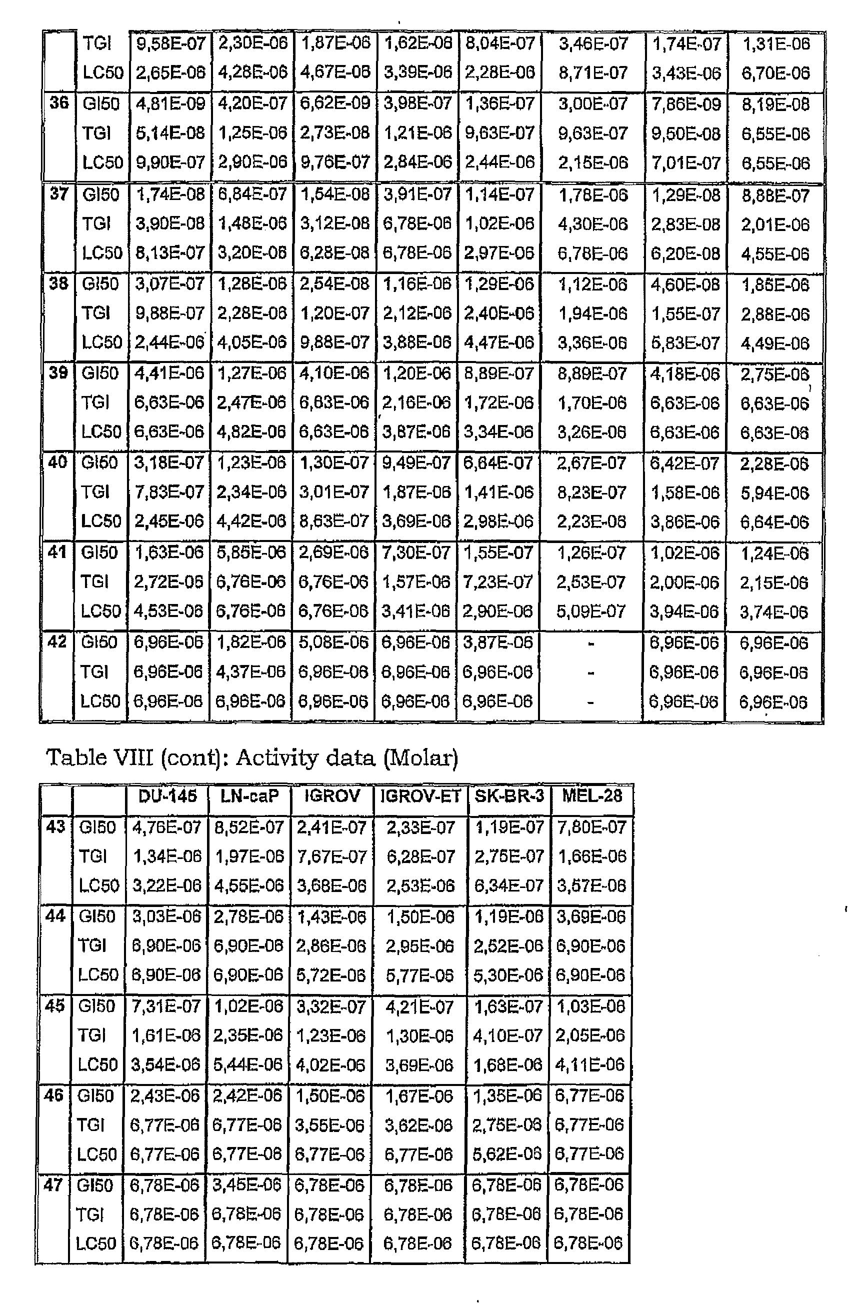

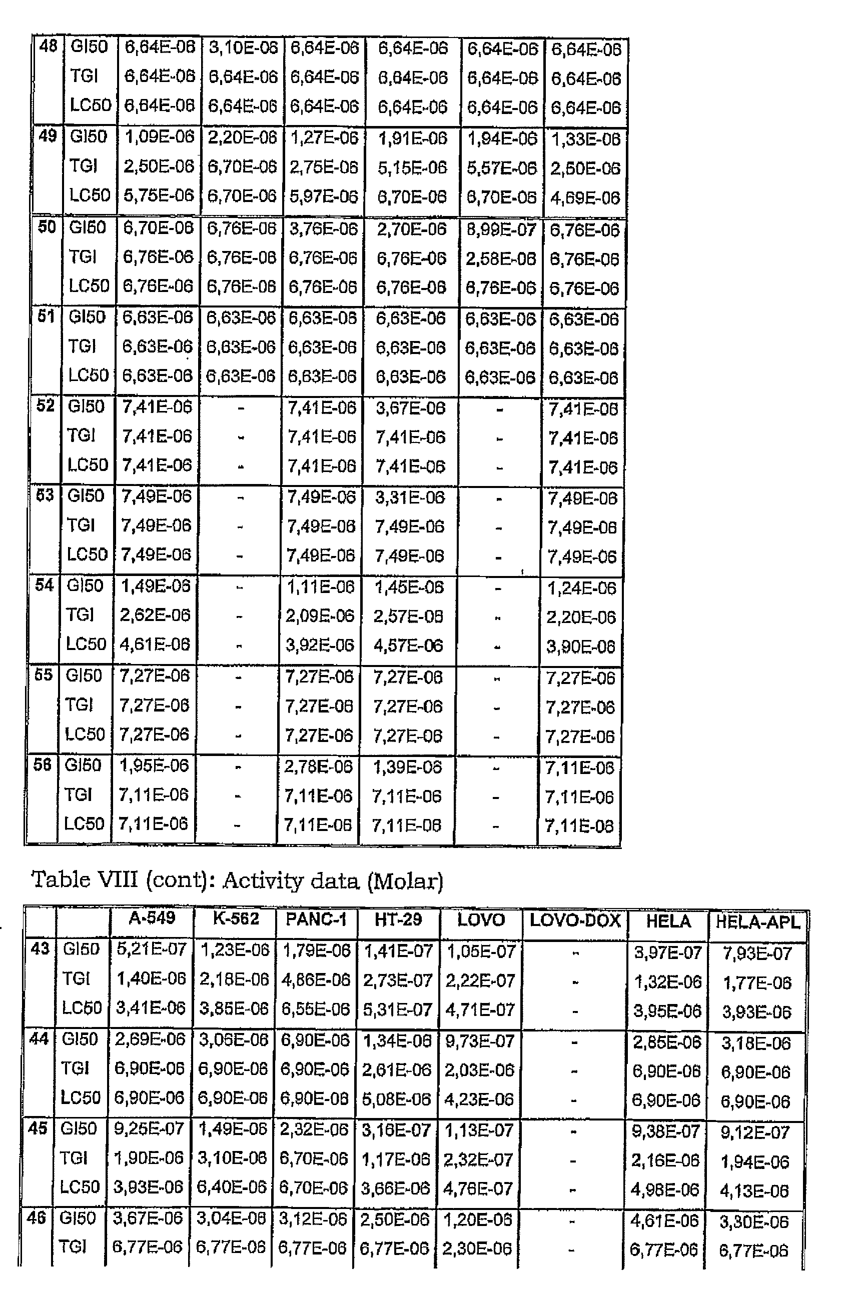

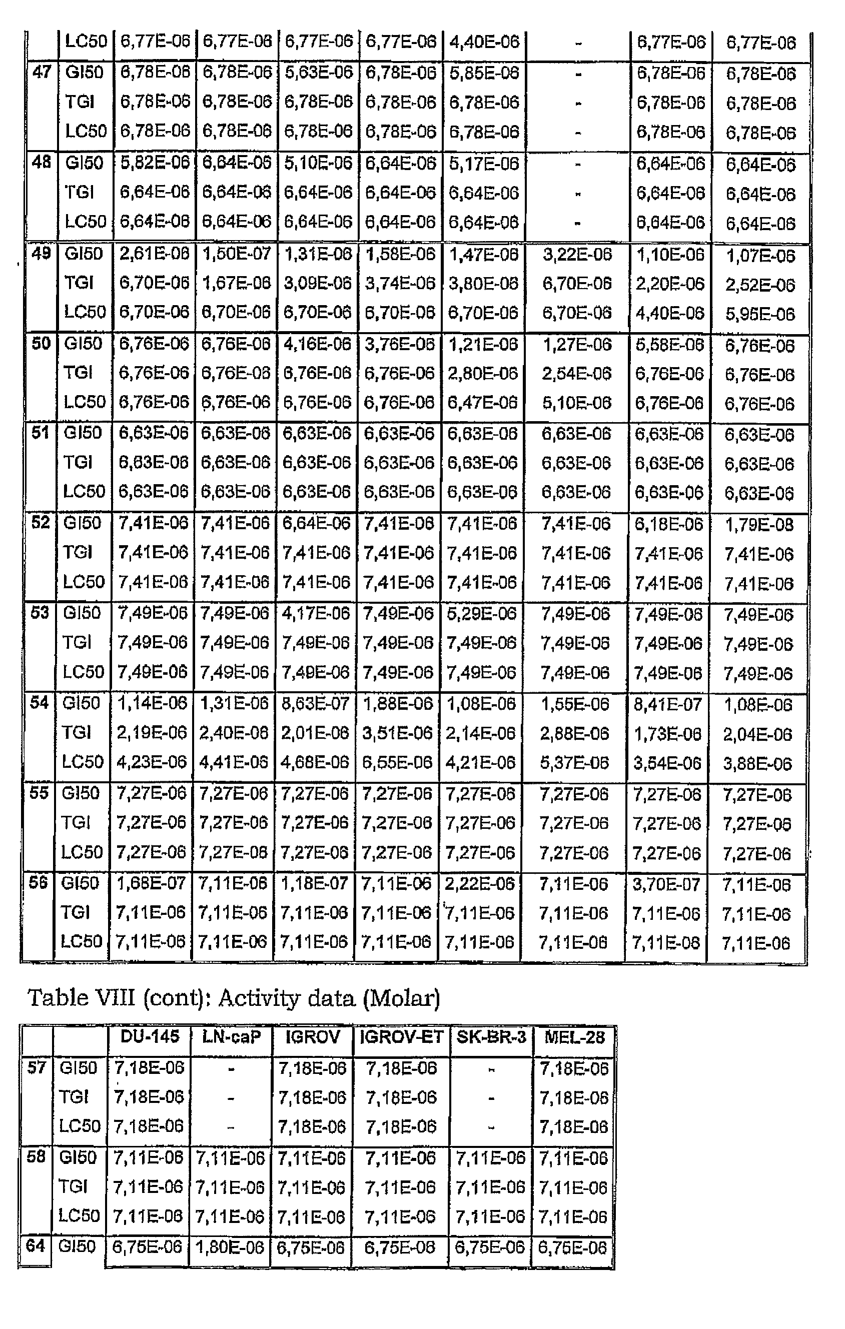

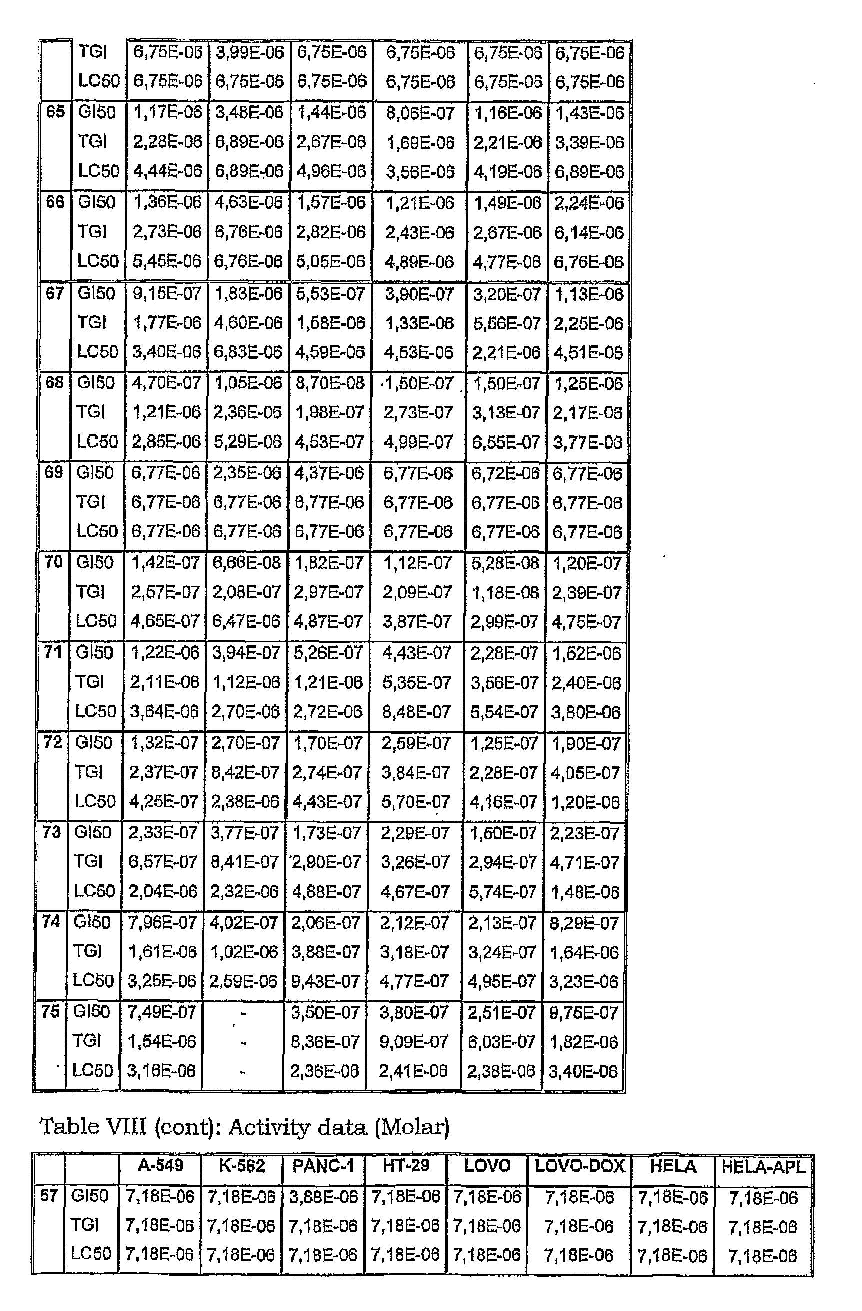

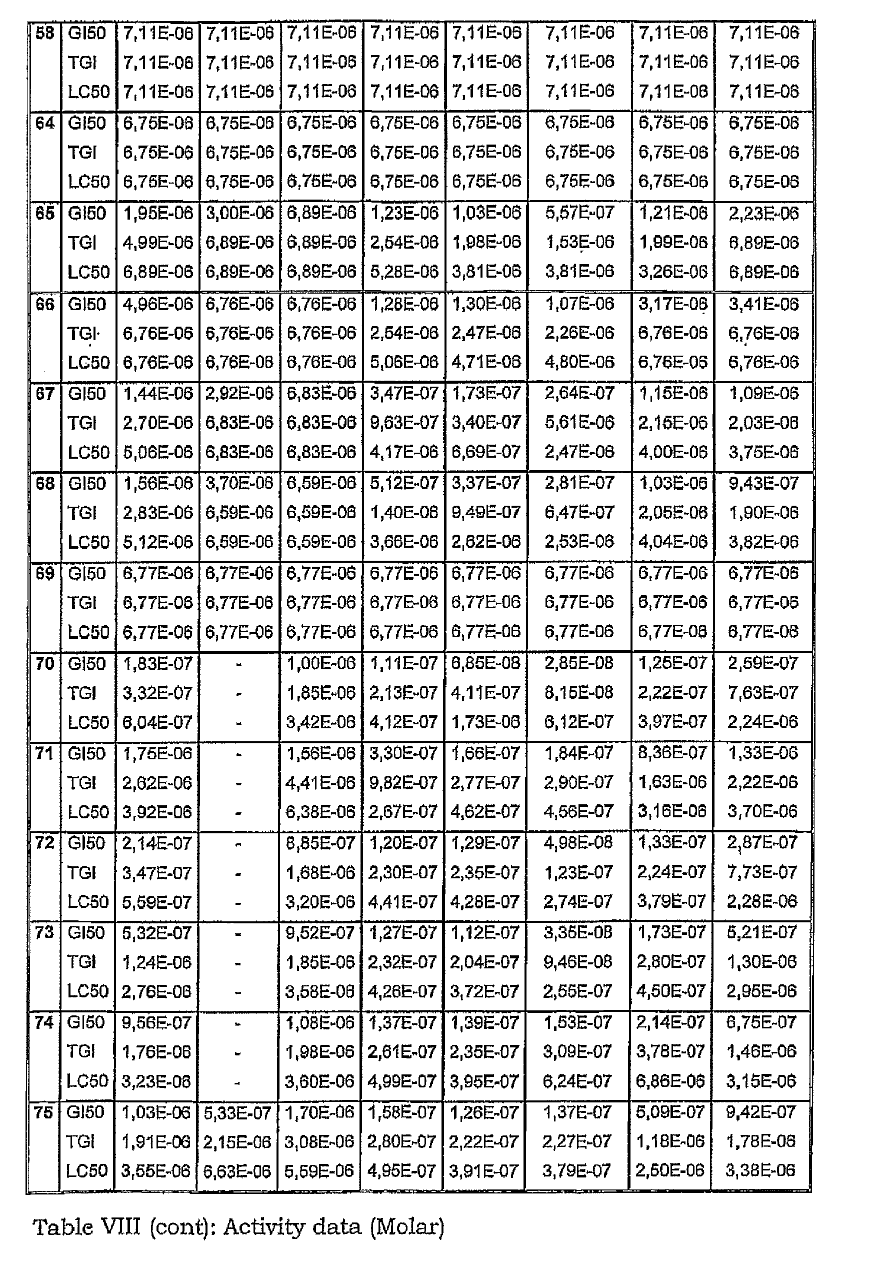

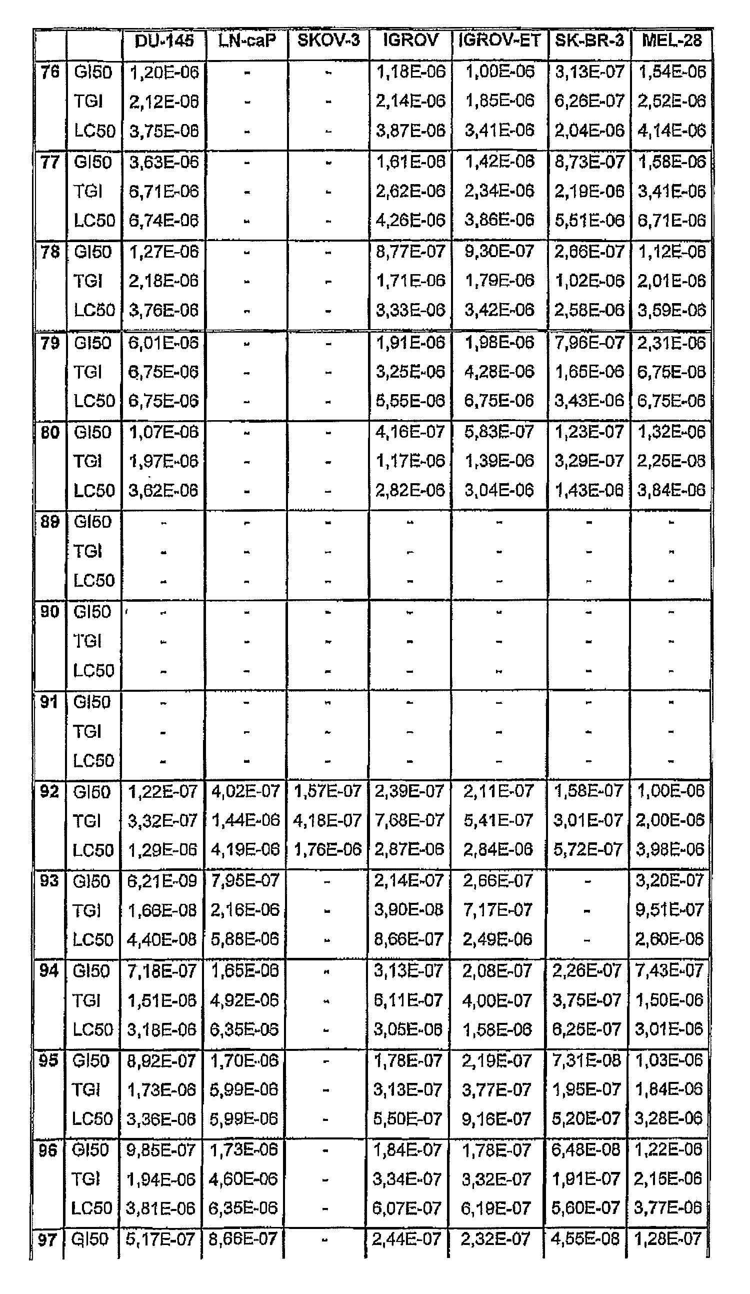

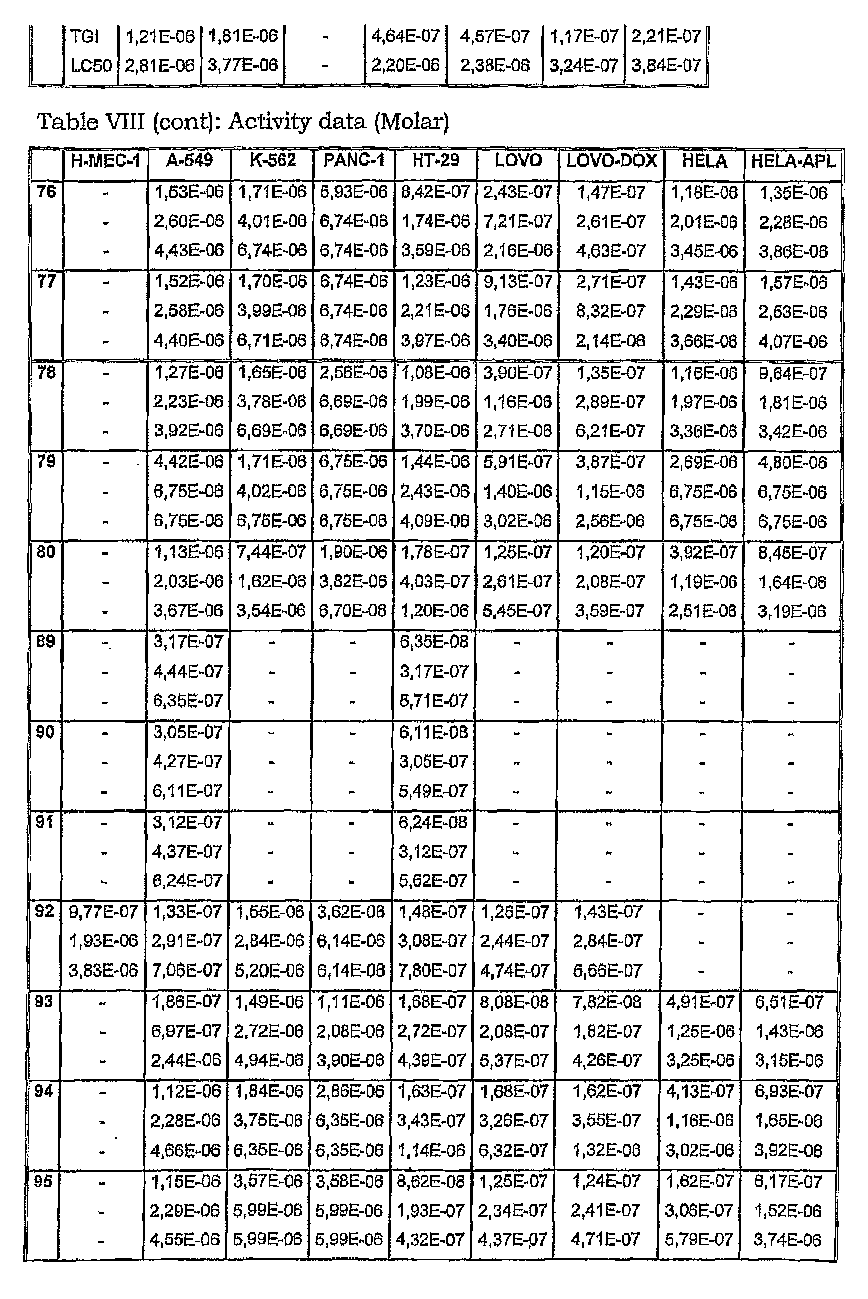

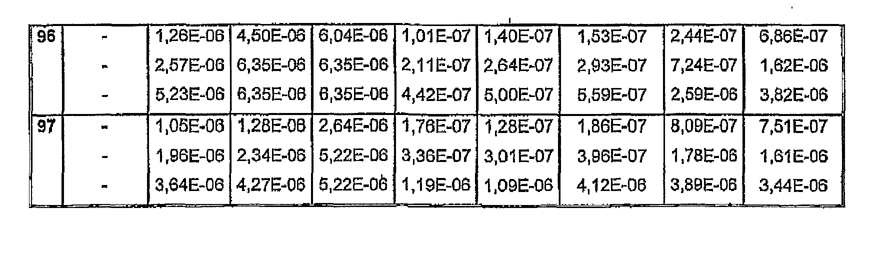

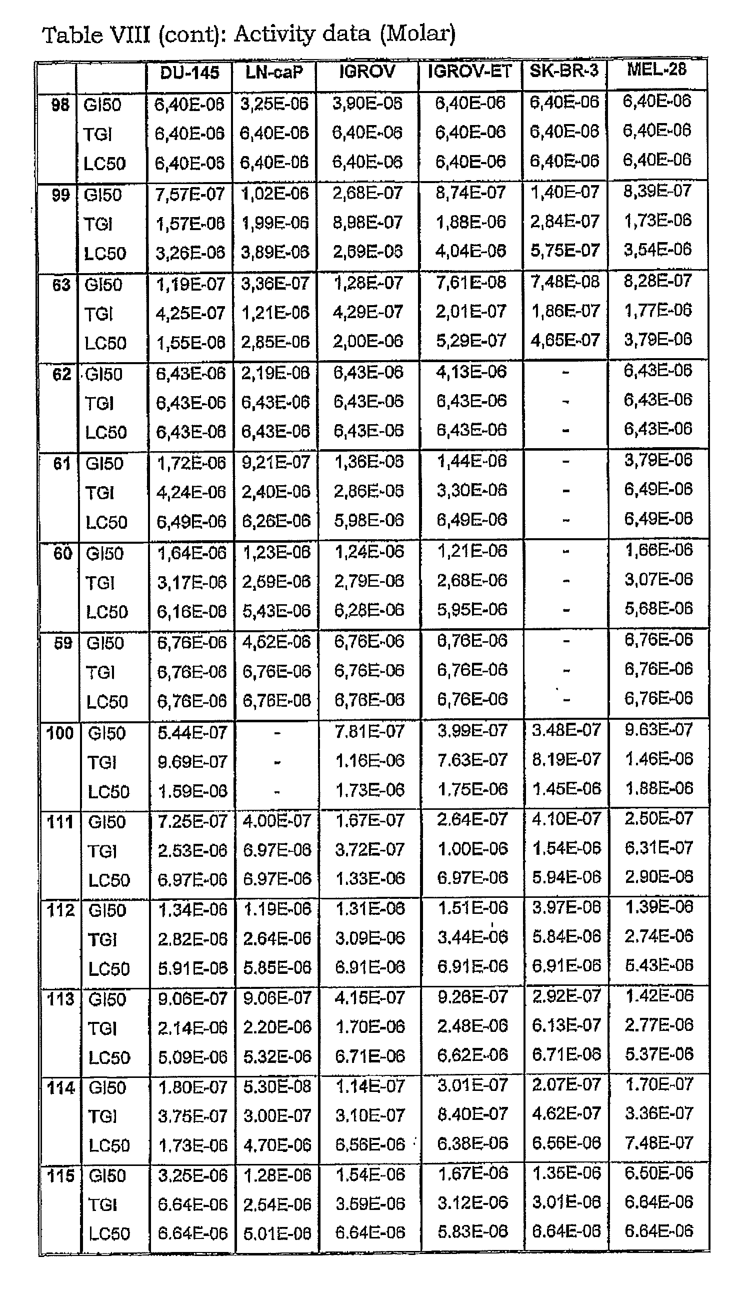

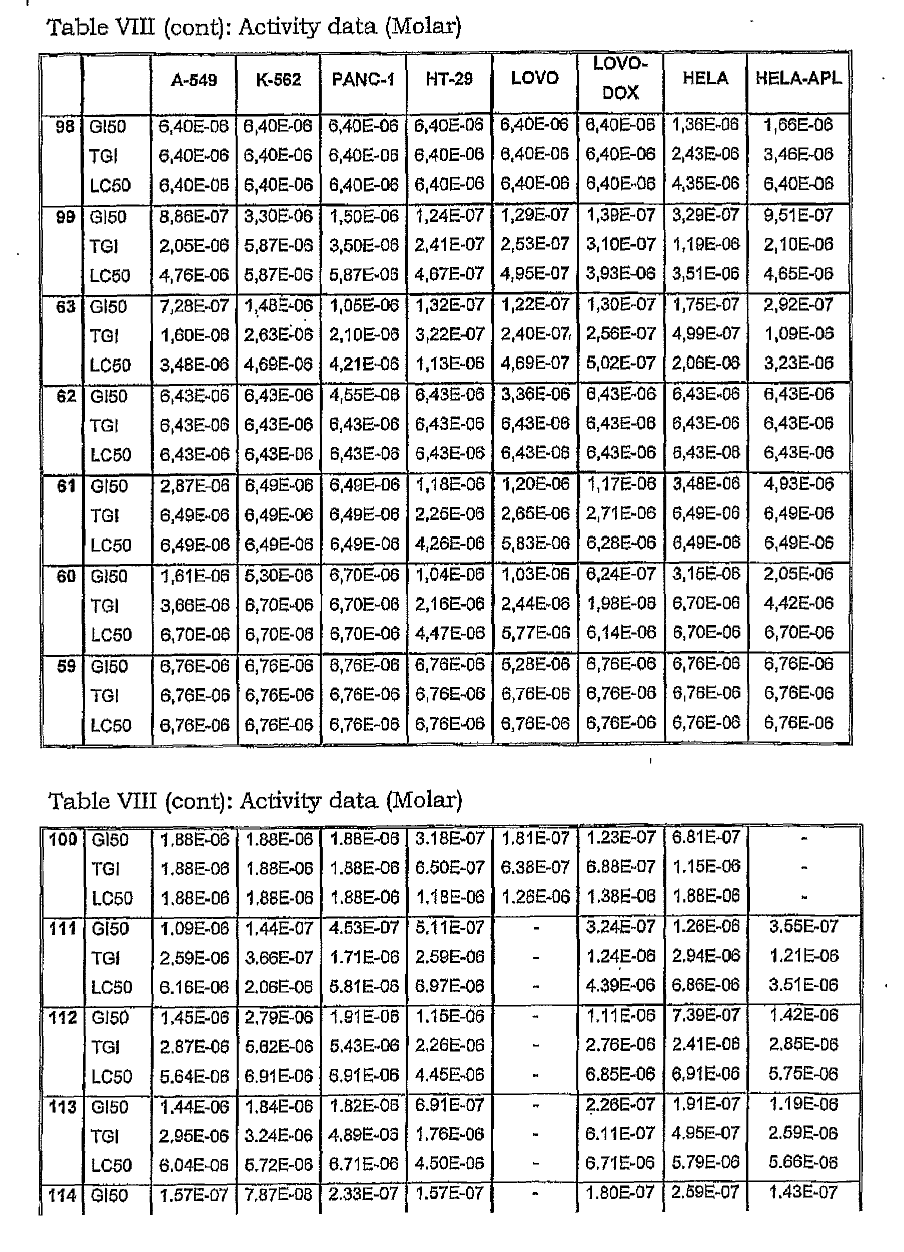

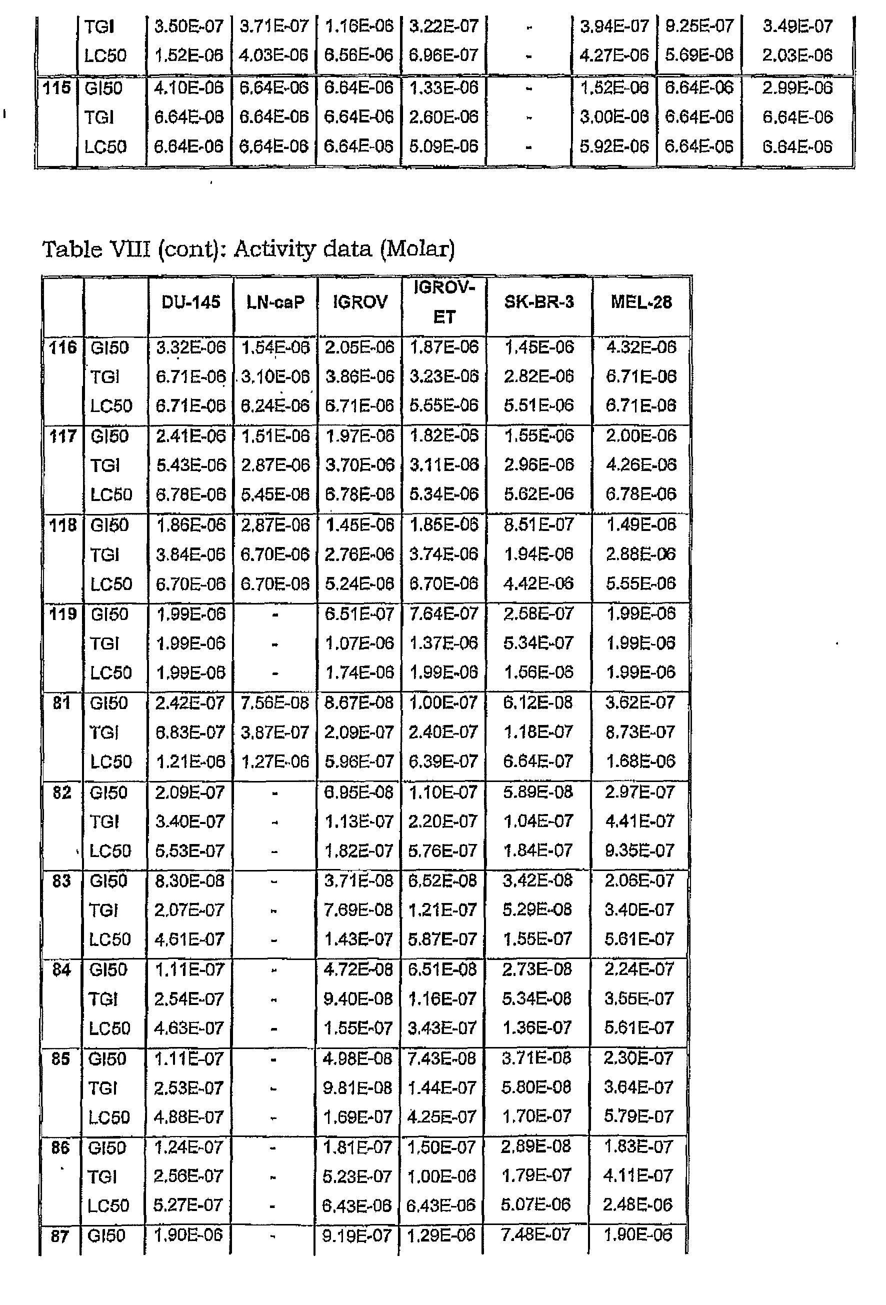

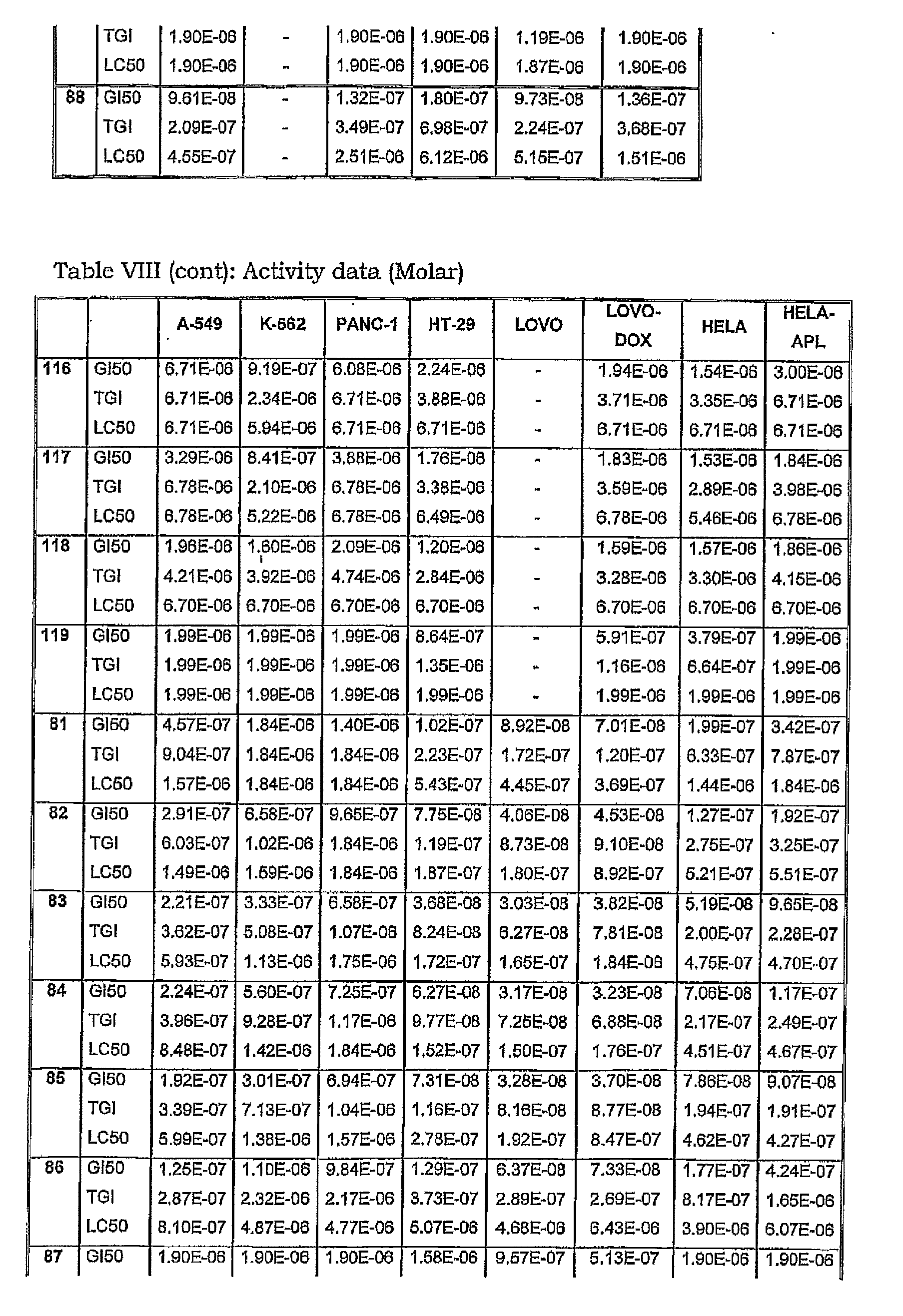

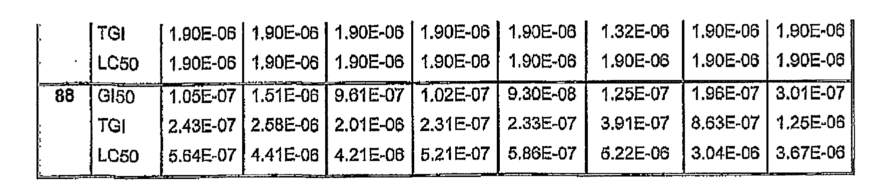

**Table IX: Activity data (Molar)**

| | | **MB-231** | **A-549** | **HT-29** |
|---|---|---|---|---|
| **101** | GI50 | 5.49E-06 | 5.49E-06 | 4.45E-06 |
| | TGI | 5.49E-06 | 5.49E-06 | 4.60E-06 |
| | LC50 | 5.49E-06 | 5.49E-06 | 4.86E-06 |
| **102** | GI50 | 5.54E-06 | 4.53E-06 | 5.54E-06 |
| | TCI | 5.54E-06 | 5.54E-06 | 5.54E-06 |
| | LC50 | 5.54E-06 | 5.54E-06 | 5.54E-06 |
| **103** | GI50 | 6.83E-06 | 6.83E-06 | 6.83E-06 |
| | TGI | 6.83E-06 | 6.83E-06 | 6.83E-06 |
| | LC50 | 6.83E-06 | 6.83E-06 | 6.83E-06 |
| **104** | GI50 | 5.09E-06 | 9.42E-07 | 3.14E-07 |
| | TGI | 5.34E-06 | 1.13E-06 | 3.39E-07 |
| | LC50 | 5.59E-06 | 1.51E-06 | 3.71E-07 |
| **105** | GI50 | 6.33E-06 | 2.28E-06 | 8.24E-07 |
| | TGI | 6.33E-06 | 2.60E-06 | 8.87E-07 |
| | LC50 | 6.33E-06 | 3.04E-06 | 8.87E-07 |
| **106** | GI50 | 6.77E-06 | 6.77E-06 | 6.77E-06 |
| | TGI | 6.77E-06 | 6.77E-06 | 6.77E-06 |
| | LC50 | 6.77E-06 | 6.77E-06 | 6.77E-06 |
| **107** | GI50 | 6.80E-06 | 6.80E-06 | 6.80E-06 |
| | TGI | 6.80E-06 | 6.80E-06 | 6.80E-06 |
| | LC50 | 6.80E-06 | 6.80E-06 | 6.80E-06 |
| **108** | GI50 | 6.53E-06 | 6.53E-06 | 6.53E-06 |
| | TGI | 6.53E-06. | 6.53E-06. | 6.53E-06. |
| | LC50 | 6.53E-06 | 6.53E-06 | 6.53E-06 |
| **109** | GI50 | 6.26E-06 | 6.26E-06 | 6.26E-06 |
| | TGI | 6.26E-06 | 6.26E-06 | 6.26E-06 |
| | LC50 | 6.26E-06 | 6.26E-06 | 6.26E-06 |
| **110** | GI50 | 3.20E-06 | 8.79E-07 | 4.08E-07 |
| | TGI | 3.71E-06 | 1.13E-06 | 5.15E-07 |
| | LC50 | 6.28E-08 | 1.63E-06 | 6.91E-07 |

## Claims

1. A compound based on the structures of kahalalide F and which differs in at least one respect.

2. A compound according to claim 1 of formula 1: 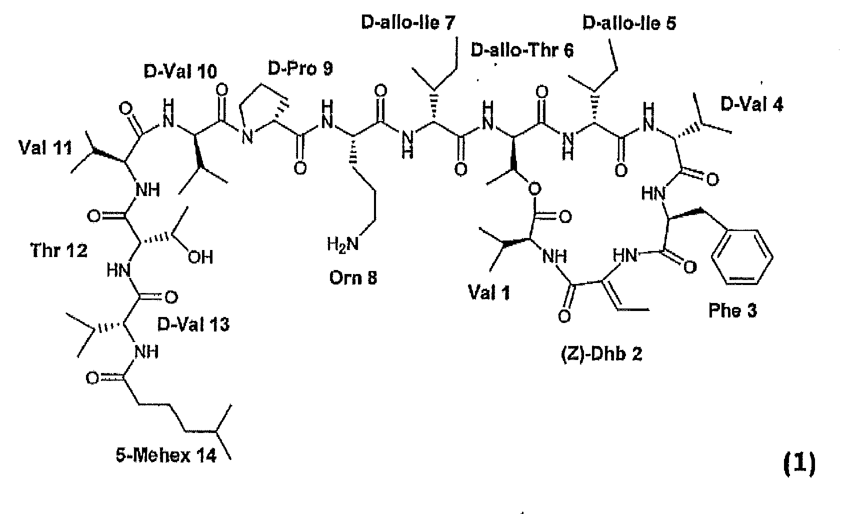 wherein one or more amino acids have been substituted by other natural or non natural amino acids, have been masked with organic groups or have been removed.

3. A compound according to claim 1 or claim 2, wherein the methylhexanoyl group has been substituted by another carboxoyl group or has been removed.

4. A compound according to claim 1, wherein an amino acid in the cyclic part is different from that in kahalalide F.

5. A compound according to claim 4, wherein the L-Phe at position 3 is changed.

6. A compound according to claim 5, wherein the amino acid at position 3 is a masked L-Phe.

7. A compound according to claim 5, wherein the amino acid at position 3 is a different natural amino acid or is a non natural amino acid.

8. A compound according to any precedent claim wherein the L-Orn at position 8 is changed.

9. A compound according to claim 1, wherein the amino acid at position 8 is a masked L-Orn.

10. A compound according to claim 5, wherein the amino acid at position 8 is a different natural amino acid or is a non natural amino acid.

11. A compound according to any preceding claim, wherein the sidechain is changed.

12. A compound according to claim 11, wherein the terminal acyl is changed.

13. A compound according to claim 12, wherein the terminal acyl is 4(S)-methylhexyl.

14. A compound according to claim 1 and based on the structure of kahalalide F of formula 1 designated KF, and selected from [Oct¹⁴]-KF, [p-MeBza¹⁴]-KF, [Bza¹⁴]-KF, [p-CF₃Bza¹⁴]-KF, [D-Thr⁶]-KF, [D-Ser⁶]-KF, [Glu⁸]-KF, [Lys⁸]-KF, [Val¹²]-KF, [D-Thr¹²]-KF, [D-Cha¹³]-KF, [hCh¹¹]-KF, [hCh¹¹, D-Cha¹³]-KF, [D-Cha⁴, D-Cha⁵,D-Cha⁷]-KF, [D-Val⁵,D-Val⁷]-KF, [Icos¹⁴]-KF, [(c/t)-4-Me-cHexa¹⁴]-KF, [Und¹⁴]-KF, [(4R)-MeHex¹⁴]-KF, [(4RS)-MeHex¹⁴]-KF, [3,5-dFPhAc¹⁴]-KF, [Pipe¹⁴]-KF, [p-CF₃Cinn¹⁴]-KF, [p-CF₃PhAc¹⁴]-KF, [Pfh¹⁴]-KF, [6-OHep¹⁴]-KF, [6,6-dFHep¹⁴]-KF, [4-GuBut¹⁴]-KF, [Lys⁸,(4S)-MeHex¹⁴]-KF,[noVal¹¹,noThr¹², noD-Val¹³]-KF, [noVal¹¹, noThr¹², noD-Val¹³, Mst¹⁴]-KF, [Gly¹³]-KF, [D-Ala¹³]-KF, [D-Leu¹³]-KF, [D-Phe¹³]-KF, [D-Pro¹³]-KF, [Val¹³]-KF, [D-Glu¹³]-KF, [D-Gln¹³]-KF, [D-Thr¹³]-KF, [Gly¹¹]-KF, [Phe¹¹]-KF, [Ala¹¹]-KF, [Leu¹¹]-KF, [D-Val¹¹]-KF, [Pro¹¹]-KF, [Gln¹¹]-KF, [Orn¹¹]-KF, [Thr¹¹]-KF, [Glu¹¹]-KF, [Ala¹², noD-Val¹3]-KF, [Gly¹², noD-Val¹³]-KF, [Leu¹², noD-Val¹³]-KF, [Pro¹², noD-Val¹³]-KF, [Glu¹², noD-Val¹³]-KF, [Orn¹², noD-Val¹³]-KF, [Gln¹², noD-Val¹³]-KF, [Pro⁹, (4S)-MeHex¹⁴]-KF, [D-Pip⁹, (4S)-MeHex¹⁴]-KF, [D-Tic⁹, (4S)-MeHex¹⁴]-KF, [(5R)-Ph-Pro⁹, (4S)-MeHex¹⁴]-KF, [hCh³]-KF, [D,L-Ser²]-KF, [Gly²]-KF, [Aib²]-KF, [Dha²]-KF, [Trp³]-KF, [D-Val¹, D-Phe³, Val⁴, allo-Ile⁵, allo-Thr⁶, allo-Ile⁷, D-Orn⁸, Pro⁹, Val¹⁰, D-Val¹¹, D-Thr¹², Val¹³] KF, [Phe(3,4-Cl₂)³, (4S)-MeHex¹⁴]-KF, [Phe(F₅)³, (4S)-MeHex¹⁴]-KF, [Phe(4-I)³, (4S)-MeHex¹⁴]-KF, [Phe(4-NO₂)³, (4S)-MeHex¹⁴]-KF, [Phe(4-F)³, (4S)-MeHex₁₄]-KF, [Tyr(Me)³, (4S)-MeHex¹⁴]-KF, [Thi³, (4S)-MeHex¹⁴]-KF, [Tic³, (4S)-MeHex¹⁴]-KF, [Tyr³, (4S)-MeHex¹⁴]-KF, [Oic³, (4S)-MeHex¹⁴]-KF, [NMePhe³, (4S)-MeHex¹⁴]-KF, [Phe(2-Cl)³]-KF, [Phe(3-Cl)³]-KF, [Phe(4-Cl)³]-KF, [Phe(3,4-F₂)³]-KF, [NaI³]-KF, [Bip³]-KF, [Phg³]-KF, [Phe(3,4-Cl₂)³, p-CF₃Cinn¹⁴]-KF, [N(Me)₂,N'(Me)₂-Arg⁸]-KF, [N(Me,Ph),N'(Me)₂-Arg⁸]-KF, [N(CH₂)₄,N'(Me)₂-Arg⁸]-KF, [N(CH₂)₄,N'(CH₂)₄-Arg⁸]-KF, [Nδ(CHN(CH₂)₄-N'(CH₂)₄)-Orn⁸]-KF, [Nε(Me)₃-Lys⁸, (4S)-MeHex¹⁴]-KF, [Thr(OTFA)¹², (4S)-MeHex¹⁴]-KF, [Orn(NδTFA)⁸, Thr(OTFA)¹², (4S)-MeHex¹⁴]-KF, [Orn(NδTFA)⁸, (4S)-MeHex¹⁴]-KF, [Thr(OTFA)¹², Lit(OTFA)¹⁴]-KF, [no 5-MeHex¹⁴,N(Hep)₂-Val¹³]-KF, [Orn(Biot)⁸]-KF, [L-Val-d₈¹¹]-KF, [AM¹⁴]-KF, [AO¹⁴]-KF, [C(=N(CH₃)₂)¹⁴]-KF, [D-Ile⁷]-KF, [D-Val⁷]-KF, [D-Orn⁸, L-Pro⁹]-KF, [D-Cys⁷, L-Cys¹¹]-KF, [D-Cys⁷, D-Cys¹⁰]-KF, [D-homoCys⁷, D-homoCys¹⁰]-KF, [Gly¹²]-KF, [Ala¹²]-KF, [Leu¹²]-KF, [Phe¹²]-KF, [Glu¹²]-KF, [Orn¹²]-KF, [Pro¹²]-KF, [D-Orn¹³]-KF, [Gln¹²]-KF and [D-Val¹]-KF,
wherein the amino acid or group indicated between brackets is the modification introduced in the structure of kahalalide F;
or a pharmaceutically acceptable salt thereof.
